# EUROPEAN PATENT APPLICATION

(11) **EP 4 053 286 A1**
(43) Date of publication of application: **07.09.2022**
(21) Application number: 21160569.6
(22) Date of filing: 03.03.2021
(51) Int. Cl.: C12N 15/90

(54) **DETERMINISTIC ORTHOGONALLY SPECIFIED INTEGRATION SITES**

(71) Applicant: Anaxon AG, 3098 Köniz (CH)
(72) Inventor: THOMET, Urs, 3018 Bern (CH)
(74) Representative: Keller Schneider Patent- und Markenanwälte AG

(57) **Abstract**

The invention comprises expression cassettes which are split in a 5'-start part and a 3'-end part. While the 5'-start part and a 3'-end part are located on different DNA strands, the expression cassette cannot be expressed. However, after successful integration, the expression cassette can be expressed and its expression indicates the success of the integration of the one DNA strand in the other. For this purpose, the 3'-end part comprises a C-centered integration specification element (CC-ISE) and a C-inner polypeptide specification element (CI-PSE). Further, the 5'-start part comprises a N-centered integration specification element (NC-ISE) and a N-inner polypeptide specification element (NI-PSE). The N-centered integration specification element (NC-ISE) and the C-centered integration specification element (CC-ISE) of the expression cassette are compatible with each other. The N-inner polypeptide specification element (NI-PSE) and the C-inner polypeptide specification element (CI-PSE) of the expression cassette are compatible with each other.

## Description

### Technical Field

The invention relates to a method to integrate genes of interest into a host genome, the DNA sequences which are used in this integration method and different applications of the integration method.

### Background Art

Biopharmaceutical production of therapeutic proteins is considered the primary prosperity booster in today's global pharmaceutical markets, accounting now for 200 USD billion in sales (Waters et al. 2019, "World Preview" 2019. EvaluatePharma 12th Edition 2019). During the past decade the advent of gene editing was driven by the disruptive discoveries around Crispr/Cas systems (Tomoko et al. 2018, "Clustered regularly interspaced short palindromic repeats (Crispr) / Crispr-associated protein 9 with improved proof-reading enhances homology-directed repair". Nucleic Acids Research 46: 4677-4688, McCarty et al. 2020, "Multiplexed CRISPR technologies for gene editing and transcriptional regulation". Nature Communications 11: 1281, Gustafsson Claes 2020, "Scientific Background on the Nobel Prize in Chemistry - A Tool for Genome Editing". The Royal Swedish Academy of Sciences, 7 October 2020). Both endeavors rely on the same idea to modify DNA code. For large-scale production of therapeutic proteins in bioreactors, living mammalian cells are genetically modified upon integration of a foreign transgene encoding the protein of interest. Gene editing aims at changing DNA for a specific purpose such as correcting a mutated gene, engineering model cells or expressing an artificial polypeptide. The conventional approach integrates plasmid-based expression cassettes for the transgene along with a marker gene (e.g. antibiotic resistance) into the host genome at a random location. Current attractive genome engineering applications include site-specific nucleases or recombinases, respectively. Site-specific nucleases including Crispr/Cas (clustered regularly interspaced short palindromic repeats), TALEN (transcription activator-like effector nucleases) and ZFN (zinc finger nucleases) constitute molecular scissors capable of recognizing target DNA sequences in order to cut them and introduce genomic double strand breaks. Most importantly, the nuclease systems inherently cannot accomplish the DNA manipulation step and therefore the endogenous DNA repair mechanism is needed to integrate foreign DNA by homologous directed repair (HDR). The HDR process is highly inefficient and rivalled by non-homologous end joining (NHEJ) to seal the introduced double strand breaks, a mechanism which actually prevents the intended DNA manipulation task. Furthermore, site-specific nucleases also cut at unintended DNA sites at frequencies actually limiting their application. Despite the disadvantages, the hype of site-specific nucleases is based on the elegant simplicity with which their target integration site can be specified or programmed. In contrast, site-specific recombinases such as Cre/LoxP or FLP/FRT are capable of accomplishing the DNA manipulation task without the endogenous DNA repair processing. The development of artificially designed RNA-guided recombinases combining the advantages of nucleases and recombinases is therefore an ongoing interest within the Crispr community (Chaikind et al. 2016, "A programmable Cas9-serine recombinase fusion protein that operates on DNA sequences in mammalian cells". Nucleic Acids Research 44: 9758-9770, Standage-Beier et al. 2019, "RNA-Guided Recombinase-Cas9 Fusion targets Genomic DNA Deletion and Integration". The Crispr Journal 2: 209-222, Hazelbaker et al. 2020, "A multiplexed gRNA piggybac transposon system facilitates efficient induction of CRISPRi and CRISPRa in human pluripotent stem cells." Scientific Reports 10: 635, Klompe et al. 2019, "Transposon encoded CRISPR-Cas systems direct RNA-guided DNA integration". Nature 571: 219-225, Kovac et al. 2020, "RNA-guided retargeting of Sleeping Beauty transposition in human cells." eLife 2020: e53868). Essentially, the success of the DNA modification step of either site-specific nucleases (indirect mechanism) or recombinases (direct mechanism) depends on the correct integration of the DNA sequence of interest. The efficiency of site-specific integration of foreign transgenes at predetermined genomic locations is generally very low and in parallel unwanted integrations at random genomic locations occur, which cannot be per se excluded. A basic challenge is that as long as the coding sequence of the selection marker remains intact, off-target expression of the marker or transgene is generally possible. This explains the impressive rate of false positive clones surviving the antibiotic selection during GMO production (Genetically Modified Organisms) by means of genome editing methods like site-specific nucleases or recombinases. In addition, gene switches and recombinase-mediated cassette exchange (RMCE) depend on the use of target recognition sites (FRT/LoxP) surrounding the coding sequence of the foreign transgene and are poorly tolerated expression-wise. For these reasons, there is a need to develop new technologies that apply stringent control over the principal recombination mechanism to effectively integrate the gene of interest into the host genome.

US2019/0078076 A1 discloses a method for knocking in a gene of interest (GOI) in a cell: According to the teaching of this document, both, the cell and the vector comprise two recombinase recognition sites (RRS) and fully functional negative selection markers with the respective complete expression cassettes. While the selection marker of the cell is surrounded by the RRS sites, the RRS sites of the vector surround the GOI. If the integration of the gene of interest is successful, the negative selection marker of the cell is replaced by the GOI and therefore elimination of all cells which do express the negative selection marker results in a set of cell where the integration was successful.

While this method allows a precise control of the integration it is restricted to negative selection. Further, the number of genes of interest which can be integrated in a cell is limited by the number of available RRS sites. In addition, it was found that RRS sites surrounding a coding sequence result in poor expression of the respective protein of interest, for example the negative selection marker or the GOI.

Pinto et al. describe in their article "An expanded library of orthogonal split intein enables modular multi-peptide assemblies" (NATURE COMMUNICATIONS, (2020) 11:1529, https://doi.org/10.1038/s41467-020-15272-2 a charactisation platform using split mCherry to evaluate known split inteins. They used the fact that non-fluorescent mCherry-intein chimeras have their fluorescence reconstituted after splicing. Therefore the fluorescence provides a fast indirect measure of cis- or trans-splicing activity of the inteins used. However Pinto et al. use standard molecular biology techniques, such as the Gibson assembly and the BioBrick RFC[10] standard to integrate the inteins to be tested at the desired location in the mCherry sequence.

Consequently, Pinto et al. do not disclose any new method for integrating a gene of interest in a host genome at all.

### Summary of the invention

It is the object of the invention to create DNA sequences pertaining to the technical field initially mentioned, which are integration sites with the property of being orthogonal and which allow a detection of a successful integration. Further, it is the object of the invention to provide such integration sites in a number which is significantly greater than the number of known RRS sites, or the number of known inteins or introns. By providing such DNA sequences, a method to integrate genes of interest into a host genome with a high degree of control as well as a great flexibility can be applied to a variety of different applications.

The solution of the invention is specified by the features of claims 1 and 2. According to the invention the DNA-sequences are a 5'-start part and a 3'-end part of an expression cassette. While the 5'-start part and a 3'-end part are located on different DNA strands, the expression cassette cannot be expressed. However, after successful integration, the expression cassette can be expressed and its expression indicates the success of the integration.

For this purpose, the 3'-end part comprises a C-centered integration specification element (CC-ISE), preferably a C-inner splicing specification element (CI-SSE) and a C-inner polypeptide specification element (CI-PSE).

Further, the 5'-start part comprises a N-centered integration specification element (NC-ISE), preferably a N-inner splicing specification element (NI-SSE) and a N-inner polypeptide specification element (NI-PSE).

According to the invention, before the integration was initiated, the 5'-start part is arranged on a DNA-strand different from the one carrying a 3'-end part.

The N-centered integration specification element (NC-ISE) and the C-centered integration specification element (CC-ISE) of the expression cassette are compatible with each other.

The N-centered integration specification element (NC-ISE) comprises a 5'-centered integrase specific integration site. The C-centered integration specification element (CC-ISE) comprises a 3'-centered integrase specific integration site.

The preferably existing N-inner splicing specification element (NI-SSE) and the preferably existing C-inner splicing specification element (CI-SSE) are compatible with each other.

Preferably, the N-inner splicing specification element (NI-SSE) is operably linked to the N-centered integration specification element (NC-ISE). The N-inner splicing specification element (NI-SSE) is itself splicing-incompetent. The N-inner splicing specification element (NI-SSE) can be rendered functional upon complementation by the C-inner splicing specification element (CI-SSE). The N-inner splicing specification element (NI-SSE) comprises a phase-specific 5'- intron sequence or a 5'- cis-intein encoding sequence.

Preferably, the C-inner splicing specification element (CI-SSE) is operably linked to the C-centered integration specification element (CC-ISE). The C-inner splicing specification element (CI-SSE) is itself splicing-incompetent. The C-inner splicing specification element (CI-SSE) can be rendered functional upon complementation by the N-inner splicing specification element (NI-SSE). The C-inner splicing specification element (CI-SSE) comprises a phase-specific 3'- intron sequence, or a 3'- cis-intein encoding sequence.

The N-inner polypeptide specification element (NI-PSE) and the C-inner polypeptide specification element (CI-PSE) of the expression cassette are compatible with each other.

In the preferred case of a 5'-start part comprising a N-inner splicing specification element (NI-SSE), the N-inner polypeptide specification element (NI-PSE) is operably linked to the N-inner splicing specification element (NI-SSE). In the case of a 5'-start part without N-inner splicing specification element (NI-SSE), the N-inner polypeptide specification element (NI-PSE) is operably linked to the N-centered integration specification element (NC-ISE).

The polypeptide encoded by the N-inner polypeptide specification element (NI-PSE) is itself non-functional. The polypeptide encoded by the N-inner polypeptide specification element (NI-PSE) can be expressed from the expression cassette upon complementation by the C-inner polypeptide specification element (CI-PSE).

In the preferred case of a 3'-end part with C-inner splicing specification element (CI-SSE), the C-inner polypeptide specification element (CI-PSE) is operably linked to the C-inner splicing specification element (CI-SSE). In the case of a 3'-end part without C-inner splicing specification element (CI-SSE), the C-inner polypeptide specification element (CI-PSE) is operably linked to the C-centred integration specification element (CC-ISE). The polypeptide encoded by the C-inner polypeptide specification element (CI-PSE) is by itself non-functional. The polypeptide encoded by the C-inner polypeptide specification element (CI-PSE) can be expressed from the expression cassette upon complementation by the N-inner polypeptide specification element (NI-PSE).

The expression cassette is essentially a nested arrangement of an integration specification element which is surrounded by a polypeptide specification element. In a preferred embodiment, the nested arrangement comprises a splicing specification element which is arranged between the integration specification element and the polypeptide specification element.

The expression cassette is split in two halves in such a way that neither the polypeptide specification element nor, in the preferred embodiment, the splicing specification element, is functional before the two halves of the expression cassette are combined in the correct sequence. Therefore the expression of the expression cassette is splicing-dependent, can be controlled upon modulation of the splicing reaction and is an indication of a correct integration. This inherently produces correct positive clones expressing one polypeptide, can accelerate the selection process and can control expression of the expression cassette upon splicing modulation.

The presence of the integration specification element, which can be any known RRS site for example, allows these DNA sequences to be integration sites. As the desired expression of the expression cassette can only occur if the integration specification element, the polypeptide specification element and preferably the splicing specification element, of the 5'-start part and the 3'-end part are compatible, a large number of orthogonal integration sites can be constructed even if only a limited number of the elements themselves are available. This can enable multiplex applications with enhanced modularity and flexibility, especially when identical RRS sites, and consequently single integrases/recombinases are used.

In the case of the preferred embodiment, it is further possible to construct a set of expression cassettes which are orthogonal, because of differences in the integration specification element or the splicing specification element, but express the same marker or produce the same polypeptide as they use the same polypeptide specification element. This can facilitate the selection process.

In the absence of a splicing specification element, the expression cassette is particularly short. In this embodiment, the N-centered integration specification element and the C-centered integration specification element are arranged within a polypeptide loop of the polypeptide specification element such that their presence does not interfere with the functionality of the polypeptide specification element in the case of a correct integration. Preferably, the location where the polypeptide specification element is divided to create the a N-inner polypeptide specification element and the C-inner polypeptide specification element is chosen such that an already present polypeptide loop at this location is extended by the N-centered integration specification element and the C-centered integration specification element, respectively. This can enable particularly short orthogonally specified integration sites.

According to the invention, an integration specification element can be any one of the following sequences: tyrosine recombinase-specific target sequences, serine recombinase-specific target sequences. N-centered integration specification elements and C-centered integration specification elements are both integration specification elements.

According to the invention, an expression cassette is termed ultrashort if the N-centered integration specification element and the C-centered integration specification element are operably linked to the respective polypeptide specification element. N-centered integration specification elements operably linked to the N-inner polypeptide specification elements and C-centered integration specification elements operably linked to the C-inner polypeptide specification elements are both ultrashort integration specification elements.

According to the invention, the term polypeptide specification element comprises polypeptide encoding DNA sequence, excluding cis-intein encoding sequence. However, a polypeptide specification element can be a trans-intein encoding sequence. N-inner polypeptide specification elements, N-outer polypeptide specification elements, C-inner polypeptide specification elements, C-outer polypeptide specification elements are all polypeptide specification elements.

According to the invention, a splicing specification element comprises inteins and/or introns. N-inner splicing specification elements, N-outer splicing specification elements, C-inner splicing specification elements, C-outer splicing specification elements are all splicing specification elements.

According to the invention, a phase-specific intron sequence describes introns, which are phase-specifically integrated into a polypeptide encoding sequence. Phase-specific 3'-intron sequences and phase-specific 5'- intron sequences are phase-specific intron sequences.

According to the invention, a cis-intein encoding sequence describes sequences encoding inteins, which are cis-splicing competent and trans-splicing incompetent, respectively. 5'-cis-intein encoding sequences, 3'- cis-intein encoding sequences are cis-intein encoding sequences. A cis-intein translates only if its 5'-encoding sequence and its 3'-encoding sequence are in the same expression cassette. A trans-intein on the other hand can be translated also if its 5'-encoding sequence and its 3'-encoding sequence are in different expression cassettes.

According to the invention, the trans-intein encoding sequences includes split-inteins, which are trans-splicing competent and cis-splicing incompetent, 5'- trans-intein encoding sequences and 3'- trans -intein encoding sequences are trans-intein encoding sequences.

According to the invention, a splicing specification element is defined as splicing-incompetent, when the element itself is not capable to undertake the splicing reaction due to the lack of the compatible splicing specification element, or the complementation with an incompatible splicing specification element.

According to the invention, a splicing specification element is defined as splicing-competent, when the element is capable to undertake the splicing reaction due to the successful complementation with a compatible splicing specification element.

According to the invention, the specification element is defined as non-functional, when the element itself is non-functional due to the lack of the compatible specification element or due to complementation with an incompatible specification element. Integration specification elements, splicing specification elements and polypeptide specification elements are specification elements.

According to the invention, the specification element is defined as functional, when the element itself is functional due to the complementation with a compatible specification element.

According to the invention, a specification element or a DNA sequence, such as the 5'-start part and the 3'-end part, is defined as incompatible, when the integration-dependent complementation by the specification element or a DNA sequence renders the expression cassette non-functional due to the complementation with an incompatible specification element or DNA sequence.

According to the invention, a specification element or a DNA sequence is defined as compatible, when the integration-dependent complementation by the specification element or the DNA sequence renders the expression cassette functional due to the complementation with a compatible specification element or DNA sequence.

According to the invention, the functional complementation of a specification element or a DNA sequence means the integration-dependent complementation of an expression cassette by a specification element or a DNA sequence, which otherwise remains non-functional due to the lack of the compatible specification element or DNA sequence, or the complementation with an incompatible specification element or DNA sequence.

In the embodiment, where the 5'-start part and the 3'-end part of an expression cassette do not comprise a N- respectively C- inner splicing specification element, the expression cassette is particularly short. Such expression cassettes are called ultrashort expression cassettes.

It was found that this embodiment works particularly well if the centered integration specification element is either an inframe linker such as an FRT site, or the centered integration specification element is completed with additional nucleotides to the next triplet. In a particular preferred embodiment, these additional nucleotides are added such that additional glycine or serine results. An example of such an addition is LoxP comprising 34 bp which is amended by the addition of two nucleotides.

Preferably, the 5'-start part and the 3'-end part of an expression cassette according to the invention are used to produce a genetically modified cell.

In this process, the strand or strands comprising the 5'-start part and at least one compatible 3'-end part are kept in a condition which favors the integration of the compatible C- and N-centered integration specification elements. A successful integration is detected by the presence of a functional expression cassette. For example, a functional expression cassette can be detected by the expression of a marker such as a fluorescence or an antibiotic resistance or by the vanishing of such a marker.

In a preferred embodiment, the 3'-end part of the expression cassette comprises a C-outer splicing specification element (CO-SSE).

In a preferred embodiment, the 5'-start part of the expression cassette comprises a N-outer splicing specification element (NO-SSE).

In these preferred embodiments, the N-outer splicing specification element (NO-SSE) and the C-outer splicing specification element (CO-SSE) are compatible with each other.

The N-outer splicing specification element (NO-SSE) is operably linked to the N-inner polypeptide specification element (NI-PSE). The N-outer splicing specification element (NO-SSE) is itself splicing-incompetent. The N-outer splicing specification element (NO-SSE) can be rendered functional upon complementation by the C-outer splicing specification element (CO-SSE). The N-outer splicing specification element (NO-SSE) comprises a 5'- cis-intein encoding sequence.

C-outer splicing specification element (CO-SSE) is operably linked to the C-inner polypeptide specification element (CI-PSE). The C-outer splicing specification element (CO-SSE) is itself splicing-incompetent. The C-outer splicing specification element (CO-SSE) can be rendered functional upon complementation by the N-outer splicing specification element (NO-SSE). The C-outer splicing specification element (CO-SSE) comprises a 3'-cis-intein encoding sequence.

The expression cassette is essentially a nested arrangement of an integration specification element which is surrounded by one polypeptide and two splicing specification elements, respectively. In a preferred embodiment, the nested arrangement comprises a polypeptide specification element which is arranged between two splicing specification elements, and wherein the outer splicing specification element comprises a cis-intein.

The expression cassette is split in two halves in such a way that neither the polypeptide specification element, in the preferred embodiment, nor the two splicing specification elements, are functional before the two halves of the expression cassette are combined in the correct sequence. Therefore the expression of the expression cassette is splicing-dependent, can be controlled upon modulation of the two splicing reactions and is an indication of a correct integration. This inherently produces correct positive clones expressing one polypeptide, can accelerate the selection process and can control expression of the expression cassette upon modulation of two splicing events.

The presence of the integration specification element, which can be any known RRS site for example, allows these DNA sequences to be integration sites. As the desired expression of the expression cassette can only occur if the integration specification element, the polypeptide specification element and preferably the two splicing specification elements, of the 5'-start part and the 3'-end part are compatible, an enhanced number of orthogonal integration sites can be specified even if only a limited number of the elements themselves are available. The two splicing specification elements enhance the degree of orthogonality of the integration specification element. This can enable further multiplex applications with enhanced modularity and flexibility, especially when identical RRS sites, and consequently single integrases/recombinases are used.

In the case of the preferred embodiment, it is further possible to construct a set of expression cassettes which are orthogonal, because of differences in the integration specification element or the two splicing specification elements, but express the same marker or produce the same polypeptide as they use the same polypeptide specification element. The two splicing specification elements enhance the degree of orthogonality of the expression cassettes. This can facilitate the selection process.

In a preferred embodiment, the 3'-end part of the expression cassette comprises a C-outer polypeptide specification element (CO-PSE).

In a preferred embodiment, the 5'-start part of the expression cassette comprises a N-outer polypeptide specification element (NO-PSE).

In this preferred embodiment, the N-outer polypeptide specification element (NO-PSE) and the C-outer polypeptide specification element (CO-PSE) are compatible with each other.

The N-outer polypeptide specification element (NO-PSE) is operably linked to the N-outer splicing specification element (NO-SSE). The polypeptide encoded by the N-outer polypeptide specification element (NO-PSE) is itself non-functional. The polypeptide encoded by the N-outer polypeptide specification element (NO-PSE) can be expressed from the expression cassette upon complementation by the C-outer polypeptide specification element (CO-PSE).

The C-outer polypeptide specification element (CO-PSE) is operably linked to the C-outer splicing specification element (CO-SSE). The polypeptide encoded by the C-outer polypeptide specification element (CO-PSE) is itself non-functional. The polypeptide encoded by the C-outer polypeptide specification element (CO-PSE) can be expressed from the expression cassette upon complementation by the N-outer polypeptide specification element (NO-PSE).

The expression cassette is essentially a nested arrangement of an integration specification element which is surrounded by two splicing and polypeptide specification elements, respectively. In a preferred embodiment, the nested arrangement comprises two polypeptide specification elements which are operably linked to two preceding splicing specification elements.

The expression cassette is split in two halves in such a way that neither the two polypeptide specification elements, in the preferred embodiment, nor the two splicing specification elements, are functional before the two halves of the expression cassette are combined in the correct sequence. Therefore the expression of the expression cassette is splicing-dependent, can be controlled upon modulation of the two splicing reactions and is an indication of a correct integration. This inherently produces correct positive clones expressing two polypeptides, can accelerate the selection process and can control expression of the expression cassette upon modulation of two splicing events.

The presence of the integration specification element, which can be any known RRS site for example, allows these DNA sequences to be integration sites. As the desired expression of the expression cassette can only occur if the integration specification element, the two polypeptide specification elements and preferably the two splicing specification elements, of the 5'-start part and the 3'-end part are compatible, a further enhanced number of orthogonal integration sites can be specified even if only a limited number of the elements themselves are available. The two polypeptide specification elements enhance the degree of orthogonality of the integration specification element. This can enable additional multiplex applications with enhanced modularity and flexibility, especially when identical RRS sites, and consequently single integrases/recombinases are used.

In the case of the preferred embodiment, it is further possible to construct a set of expression cassettes which are orthogonal, because of differences in the integration specification element or the two splicing specification elements, but express the same two markers or produce the same two polypeptides as they use the same two polypeptide specification elements. The two polypeptide specification elements enhance the degree of orthogonality of the expression cassettes. This can facilitate the selection process.

In a preferred embodiment, the C-inner splicing specification element (CI-SSE) comprises two splicing specifications which are the phase-specific 3'- intron sequence and the 3'- cis-intein encoding sequence.

The phase-specific 3'- intron sequence and the 3'- cis-intein encoding sequence are examples of splicing specifications which are modulatory.

By increasing the number of modulatory splicing specifications in the C-inner splicing specification element (CI-SSE), the number of available C-inner splicing specification elements (CI-SSE) is increased and the splicing-dependent expression of the expression cassette can be controlled by two different splicing reactions.

There are two ways to arrange the two splicing specifications in this embodiment, respectively. Either, the phase-specific 3'- intron sequence is upstream of the 3'- cis-intein encoding sequence or the phase-specific 3'- intron sequence is downstream of the 3'- cis-intein encoding sequence.

The case of the N-inner splicing specification element (NI-SSE) of the expression cassette comprising two splicing specifications, too, is discussed in more detail below.

In one embodiment of expression cassettes, the N-inner splicing specification element (NI-SSE) comprises only one splicing specification which is compatible with only one out of two splicing specifications of the C-inner splicing specification element (CI-SSE).

This fact can be used to prevent the expression of the polypeptide specification element in the case of a successful integration while allowing the expression in the case of a wrong integration or if no integration took place at all. In order to do so, the N-inner splicing specification element (NI-SSE) comprises a splicing specification compatible with the upstream splicing specification of the C-inner splicing specification element (CI-SSE). In this way the downstream splicing specification remains as between the N-inner polypeptide specification element and the C-inner polypeptide specification element after the respective splicing event upon successful integration. The downstream splicing specification is thereby either an interposed exonic intein or an exteinic inframe intron which interferes with the expression of the polypeptide of the expression cassette. However, if the strand carrying the C-inner splicing specification element (CI-SSE) carries a downstream splicing specification which is compatible to the N-inner splicing specification element, this a downstream splicing specification will be spliced, if the integration fails. This splicing results in the expression of the respective polypeptide. The downstream splicing specification is either the intronic intein or inteinic inframe intron. Therefore an expression of the polypeptide of the expression cassette is an indication for a failed integration in this embodiment.

In a preferred embodiment of an expression cassette, the N-inner splicing specification element (NI-SSE) comprises the phase-specific 5'- intron sequence downstream of the 5'-cis-intein encoding sequence. In this expression cassette, the 3'- cis-intein encoding sequence is arranged downstream of the phase-specific 3'- intron sequence.

In this embodiment, the splicing specification element of the expression cassette allows and requires both: mRNA splicing and protein splicing. Therefore, the number of available inner splicing specification elements increases. Further, as two splicing processes are needed for an expression of the polypeptide specification element, the risk for a false positive signal is reduced. The splicing-dependent expression of the expression cassette can be controlled by two different splicing reactions. As the polypeptide specification element is only expressed if both splicing events occur, an expression cassette according to this embodiment may be used as logical AND-gate with the conditions for intein and intron splicing being the input and the expression of the polypeptide specification element being the output.

In a preferred embodiment of an expression cassette, the N-inner splicing specification element (NI-SSE) comprises two splicing specifications which are the phase-specific 5'-intron sequence and the 5'- cis-intein encoding sequence.

The phase-specific 5'- intron sequence and the 5'- cis-intein encoding sequence are examples of splicing specifications which are modulatory.

There are two ways to arrange the two splicing specifications of this embodiment: Either, the phase-specific 5'- intron sequence is upstream of the 5'- cis-intein encoding sequence or the phase-specific 5'- intron sequence is downstream of the 5'- cis-intein encoding sequence.

Particularly preferably, the C-inner splicing specification element (CI-SSE) of this expression cassette comprises only one compatible splicing specification. The C-inner splicing specification element (CI-SSE) comprises a splicing specification which is compatible with the downstream splicing specification of the N-inner splicing specification element (NI-SSE).

In this embodiment, a splicing logic which is analogous to the splicing logic of the case in which the C- inner splicing specification element comprises two splicing specification and the N-inner splicing specification element comprises only one compatible splicing specification, is possible.

A host strand according to this invention is a DNA-strand comprising a 5'-start part of an expression cassette.

A host strand of DNA according to this invention comprises preferably two or more DNA-sequences which are 5'-start parts of different expression cassettes.

The location of the 5'-start part defines the location to integrate a gene of interest in the host strand.

The presence of different 5'-start parts at different locations on the host strand allows to control the integration of different genes at different locations simultaneously in a controlled manner.

In a preferred embodiment, the host strand comprises a 3'-end part which is located downstream of the 5'-start part. The 3'-end part and the 5'-start part are incompatible which each other. The 3'-end part and the 5'-start part belong to different expression cassettes.

In a particularly preferred embodiment, the 5'-centred integrase specific integration site and the 3'-centred integrase specific integration site are orthogonal to each other and/or the C-inner splicing specification element of the 3'-end part is not compatible with the N-inner splicing specification element of the 5'-start part.

In a further particularly preferred embodiment, the 5'-centred integrase specific integration site and the 3'-centred integrase specific integration site are orthogonal to each other and the C-inner splicing specification element of the 3'-end part is not compatible with the N-inner splicing specification element of the 5'-start part. This embodiment ensures a correct integration by two independent mechanisms: The orthogonality of the integration sites and the possibility to express the polypeptide which is in this case only possible if the splicing occurs as intended.

Orthogonal integration sites are characterized in that a given 3'-centred integrase specific integration site is not compatible with an orthogonal 5'-centred integrase specific integration site and given 5'-centred integrase specific integration site is not compatible with an orthogonal 3'-centred integrase specific integration site.

Essentially, the 5'-centred integrase specific integration site and the 3'-centred integrase specific integration site delimit a section of the host strand which can be replaced by the compatible integration strand. Therefore, such a host strand can be used for cassette exchange procedures and mutagenesis. Furthermore, in this configuration the integration specification elements are well tolerated expression-wise.

In a preferred embodiment of a host strand, the N-inner splicing specification element (NI-SSE) and the C-inner splicing specification element (CI-SSE) are incompatible with each other and /or the N-inner polypeptide specification element (NI-PSE) and the C-inner polypeptide specification element (CI-PSE) are incompatible with each other.

This has the advantage that the polypeptide specification element cannot be expressed in the absence of an integration of the compatible integration strand.

In a preferred embodiment of a host strand comprising a 3'-end part arranged downstream to a compatible the 5'-start part. In this embodiment the N-inner splicing specification element (NI-SSE) and the C-inner splicing specification element (CI-SSE) are compatible with each other and the N-inner polypeptide specification element (NI-PSE) and the C-inner polypeptide specification element (CI-PSE) are compatible with each other.

This has the advantage that the polypeptide specification element is expressed in the absence of an integration of the compatible integration strand and ceases to be expressed when an integration strand, preferably carrying an compatible N-inner splicing specification element (NI-SSE) and an compatible C-inner splicing specification element (CI-SSE) but an incompatible N-inner polypeptide specification element (NI-PSE) and an incompatible C-inner polypeptide specification element (CI-PSE).

In a preferred embodiment of a host strand the 5'-start part comprises the N-outer splicing specification element (NO-SSE) and the N-outer polypeptide specification element (NO-PSE) and a 3'-end part arranged downstream on this host strand comprises the C-outer splicing specification element (CO-SSE) and the C-outer polypeptide specification element (CO-PSE).

In this embodiment, the N-inner splicing specification element (NI-SSE) and the C-inner splicing specification element (CI-SSE), the N-inner polypeptide specification element (NI-PSE) and the C-inner polypeptide specification element (CI-PSE), the N-outer splicing specification element (NO-SSE) and the C-outer splicing specification element (CO-SSE) as well as the N-outer polypeptide specification element (NO-PSE) and the C-outer polypeptide specification element (CO-PSE) are compatible with each other.

In this embodiment, the N-inner splicing specification element (NI-SSE) comprises the phase-specific 5'- intron sequence and the C-inner splicing specification element (CI-SSE) comprises the phase-specific 3'-intron sequence.

In this embodiment, the intron splicing brings the inner polypeptide specification elements in contact with each other and allows for example the expression of a desired polypeptide. This can be used to generate a variant library of the gene of interest within a region of the coding sequence actually specified by the 5'-centred integrase specific integration site and the 3'-centred integrase specific integration site. The intein splicing however, brings the outer polypeptide specification elements in contact with each other and allows for example the expression of a marker.

An integration strand is a DNA strand. It comprises a 3'-end part and preferably one or more genes of interest to be integrated in a host strand.

In a preferred embodiment, the integration strand comprises further a 5'-start part.

In a preferred embodiment, the genes of interest to be integrated in the host strand form the C-inner polypeptide specification element at least partially .

In a preferred embodiment, an integration strand comprisesa 5'-start part and a 3'-end part. One or more genes of interest are in this embodiment arranged between the 5'-start part and the 3'-end part. Preferably, the 5'-start part and the 3'-end part are incompatible with each other.

In a particularly preferred embodiment of an integration strand, the 5'-centred integrase specific integration site and the 3'-centred integrase specific integration site are orthogonal to each other and/or the C-inner splicing specification element of the 3'-end part is not compatible with the N-inner splicing specification element of the 5'-start part.

The use of two incompatible integration sites allows to use the concept of the invention in cassette exchange procedures as the different integration sites mark the beginning and the end of the sequences to be exchanged. Furthermore, in this configuration the integration specification elements are well tolerated expression-wise.

In a preferred embodiment, the integration strand comprises a first DNA-sequence and a second DNA-sequence. The second DNA-sequence is a 3'-end part.

The first DNA sequence comprises a N-inner splicing specification element (NI-SSE). The N-inner splicing specification element is compatible with a C-inner splicing specification element (CI-SSE). The N-inner splicing specification element is itself splicing-incompetent. The N-inner splicing specification element (NI-SSE) can be rendered functional upon complementation by the C-inner splicing specification element (CI-SSE). The N-inner splicing specification element (NI-SSE) comprises a phase-specific 5'- intron sequence, or a 5'- cis-intein encoding sequence.

The first DNA sequence comprises further a N-inner polypeptide specification element (NI-PSE). The N-inner polypeptide specification element (NI-PSE) is compatible with a C-inner polypeptide specification element (CI-PSE). The N-inner polypeptide specification element (NI-PSE) is operably linked to the N-inner splicing specification element (NI-SSE). A polypeptide encoded by the N-inner polypeptide specification element (NI-PSE) is itself non-functional. A polypeptide encoded by the N-inner polypeptide specification element (NI-PSE) can be expressed from the expression cassette upon complementation by the C-inner polypeptide specification element (CI-PSE).

The C-centered integration specification element (CC-ISE) on the integration strand comprises preferably a tyrosine integrase system.

In this preferred embodiment, one or more genes of interest are arranged between the first and the second DNA-sequence.

As there is only a single integration specification element (CC-ISE) on the integration strand, multiple integration strands can be integrated downstream and adjacent to each other at the same position of the host strand using only a single integrase system, which is preferably a tyrosine integrase system.

Preferably, the N-inner splicing specification element of the first DNA sequence is operably linked to the C-centered integration specification element (CC-ISE) of the second DNA sequence.

In another embodiment, the integration strand comprises a first DNA-sequence and a second DNA-sequence. The first DNA-sequence is a 5'-start part.

The second DNA sequence comprises a C-inner splicing specification element (CI-SSE). The C-inner splicing specification element is compatible with a N-inner splicing specification element (NI-SSE). The C-inner splicing specification element is itself splicing-incompetent. The C-inner splicing specification element (CI-SSE) can be rendered functional upon complementation by the N-inner splicing specification element (NI-SSE). The C-inner splicing specification element (CI-SSE) comprises a phase-specific 3'- intron sequence, or a 3'- cis-intein encoding sequence.

The second DNA sequence comprises further a C-inner polypeptide specification element (CI-PSE). The C-inner polypeptide specification element (CI-PSE) is compatible with a N-inner polypeptide specification element (NI-PSE). The C-inner polypeptide specification element (CI-PSE) is operably linked to the C-inner splicing specification element (CI-SSE). A polypeptide encoded by the C-inner polypeptide specification element (CI-PSE) is itself non-functional. A polypeptide encoded by the C-inner polypeptide specification element (CI-PSE) can be expressed from the expression cassette upon complementation by the N-inner polypeptide specification element (NI-PSE).

The N-centered integration specification element (NC-ISE) on the integration strand comprises preferably a tyrosine integrase system.

In this preferred embodiment, one or more genes of interest are arranged between the first and the second DNA-sequence.

This embodiment differs from the previous embodiment only by the fact that there is a N-centered integration specification element (NC-ISE) instead of a C-centered integration specification element (CC-ISE).

Preferably, the N-centered integration specification element (NC-ISE) and the C-centered integration specification element (CC-ISE) are identical. Preferably, the C-inner splicing specification element of the second DNA sequence is operably linked to the N-centered integration specification element (NC-ISE) of the first DNA sequence.

Upon use of more than two integration strands according to this or the previous embodiment, multiple integration strands can be integrated downstream and adjacent to each other at the same position of the host strand using only a single integrase.

In a preferred embodiment of an integration strand comprising a first and a second DNA sequence and either a 3'-end part or a 5'-start part, the C-inner splicing specification element (CI-SSE) of the second DNA-sequence comprises a first 3'-inner splicing specification upstream of a second 3'-inner splicing specification. According to this embodiment, the N-inner splicing specification element (NI-SSE) of the first DNA-sequence is compatible to the second 3'-inner splicing specification.

In a first version of this preferred embodiment, the first 3'-inner splicing specification is a 3'-cis-intein encoding sequence and the second 3'-inner splicing specification is a phase-specific 3'- intron sequence and the N-inner splicing specification element (NI-SSE) is the compatible phase-specific 5'- intron sequence.

In a second version of this preferred embodiment, the first 3'-inner splicing specification is a phase-specific 3'- intron sequence and the second 3'-inner splicing specification is a 3'-cis-intein encoding sequence and the N-inner splicing specification element (NI-SSE) is the compatible 5'- cis-intein encoding sequence.

Preferably and in both versions, the N-inner polypeptide specification element (CI-PSE) of the first DNA-sequence and the C-inner polypeptide specification element (CI-PSE) of the second DNA-sequence are compatible with each other.

These embodiments allow to integrate sequences of integration strands downstream and adjacent to each other whereby the integration strands in this sequence alternate between the first and the second version.

If the polypeptide specification elements are compatible with each other, integration strands which are not integrated in a host strand and integration strands which are integrated in a wrong way can express the polypeptide. Consequently, the lack of an expression is a signal for a successful integration in this embodiment.

In a preferred embodiment of an integration strand comprising a first and a second DNA sequence and either a 3'-end part or a 5'-start part, the N-inner splicing specification element (NI-SSE) of the first DNA-sequence comprises a first 5'-inner splicing specification followed by a second 5'-inner splicing specification. According to this embodiment, the C-inner splicing specification element (CI-SSE) of the second DNA-sequence is compatible to the first 5'-inner splicing specification.

In a first version of this preferred embodiment, the first 5'-inner splicing specification is a 5'-cis-intein encoding sequence and the second 5'-inner splicing specification is a phase-specific 5'- intron sequence and the C-inner splicing specification element (CI-SSE) is the compatible 3'- cis-intein encoding sequence.

In a second version of this preferred embodiment, the first 5'-inner splicing specification is a phase-specific 5'- intron sequence and the second 5'-inner splicing specification is a 5'-cis-intein encoding sequence and the C-inner splicing specification element (CI-SSE) is the compatible phase-specific 3'- intron sequence.

Preferably and in both versions, the N-inner polypeptide specification element (CI-PSE) of the first DNA-sequence and the C-inner polypeptide specification element (CI-PSE) of the second DNA-sequence are compatible with each other.

The splicing logic of these embodiments is analogous to the embodiments, where the C-inner splicing specification element (CI-SSE) of the second DNA-sequence comprises two splicing specifications.

In one embodiment of an integration strand comprising a first and a second DNA sequence and a 3'-end part, the first DNA-sequence comprises a N-outer splicing specification element (NO-SSE) and a N-outer polypeptide specification element (NO-PSE). The 3'-end part of this embodiment omprises a C-outer splicing specification element (CO-SSE) and a C-outer polypeptide specification element (CO-PSE).

In this embodiment the N-outer splicing specification element (NO-SSE) is compatible with the C-outer splicing specification element (CO-SSE). The N-outer splicing specification element (NO-SSE) is operably linked to the N-inner polypeptide specification element (NI-PSE). The N-outer splicing specification element (NO-SSE) is itself splicing-incompetent. The N-outer splicing specification element (NO-SSE) can be rendered functional upon complementation by a C-outer splicing specification element (CO-SSE). The N-outer splicing specification element (NO-SSE) comprises a 5'- cis-intein encoding sequence.

In this embodiment, the N-outer polypeptide specification element (NO-PSE) is compatible with the C-outer polypeptide specification element (CO-PSE). The N-outer polypeptide specification element (NO-PSE) is linked to the N-outer splicing specification element (NO-SSE). A polypeptide encoded by the N-outer polypeptide specification element (NO-PSE) is by itself non-functional and can be expressed from the expression cassette upon complementation by the C-outer polypeptide specification element (CO-PSE).

In this embodiment, the C-outer splicing specification element (CO-SSE) and the N-outer splicing specification element are compatible. Further, the C-outer polypeptide specification element (CO-PSE) and the N-outer polypeptide specification element (NO-PSE) are compatible and, result, in the case of successful splicing, in the expression of an outer marker.

In this case, a falsely integrated or unintegrated integration strand can express the polypeptide encoded by the N- and C-outer polypeptide specification element. This expression is inhibited when the integration strand is integrated correctly. Further, the polypeptides partially encoded by the N- and C-inner polypeptide specification elements which are compatible with N- and C-inner polypeptide specification elements arranged up- and downstream of the integration strand can, in case of a correct integration, be expressed and indicate thereby a successful integration. Consequently, integration strands according to this embodiment can indicate with a first marker a successful integration and with a second marker a false integration.

In a first set of a host strand and an integration strand, the host strand and the integration strand have the following properties:
The host strand of this first set comprises a 5'-start part defining an integration site.

The 5'-start part comprises the N-inner polypeptide specification element, the N-inner splicing specification element (NI-SSE) and the N-centered integration specification element.

N-centered integration specification element comprises a 5'-centered integrase specific integration site.

The N-inner polypeptide specification element is is operably linked to the N-inner splicing specification elements (NI-SSE). The N-inner splicing specification element (NI-SSE) is operably linked to the N-centered integration specification element

The N-inner splicing specification elements (NI-SSE) made of the 5'- cis-intein encoding sequence arranged upstream and adjacent to the phase-specific 5'- intron sequence.

The N-inner polypeptide specification element can express a marker when completed with a compatible C-inner polypeptide specification element.

The integration strand comprises a 3'-end part.

The 3'-end part comprises the C-centered integration specification element, the C-inner splicing specification elements (CI-SSE) and the C-inner polypeptide specification element.

The C-centered integration specification element comprises the 3'-centered integrase specific integration site. The 3'-centered integrase specific integration site is compatible with the 5'-centered integrase specific integration site on the host strand.

The C-inner polypeptide specification element is operably linked to the C-inner splicing specification elements (CI-SSE). The C-inner splicing specification element (CI-SSE) is operably linked to the C-centered integration specification element.

The C-inner splicing specification element (CI-SSE) is made of the phase-specific 3'- intron sequence arranged adjacent to and upstream of the 3'- cis-intein encoding sequence. The phase-specific 3'- intron sequence is compatible with the phase-specific 5'- intron sequence on the host strand, The 3'- cis-intein encoding sequence is compatible with the 5'- cis-intein encoding sequence on the host strand.

The C-inner polypeptide specification element of the integration strand can express a marker when completed with the compatible N-inner polypeptide specification element of the host strand.

In this embodiment, the integration strand comprises the gene of interest to be integrated in the host strand.

This first set is preferably used to prevent undesired off-target integration, as the marker can only be expressed if both, the intein and the intron splicing occurs correctly. Therefore, the expression of the marker occurs only if the integration strand is integrated at the integration site. Furthermore, the splicing-dependent expression of the expression cassette can be controlled by two different splicing reactions.

The first set is preferably used as a logical AND gate, whereby a first trigger, which can cause the Intron splicing, and a second trigger, which can cause the intein splicing, are the input signals and the expression of the marker is the output signal. The marker is only expressed if both input signals were given.

In a second set of a host strand and two or more integration strands, which should be integrated in the host strand in a desired sequence, the host strand and the integration strands have the following properties:
The host strand comprises a 5'-start part. The 5'-start part comprises the N-inner polypeptide specification element, the N-inner splicing specification element (NI-SSE) and the N-centered integration specification element.

The N-inner polypeptide specification element (NI-PSE) is operably linked to the N-inner splicing specification element (NI-SSE). The N-inner splicing specification element (NI-SSE) is operably linked to the N-centered integration specification element (NC-ISE).

The N-inner polypeptide specification element is compatible with the C-inner polypeptide specification element of the following integration strand. The N-inner splicing specification element (NI-SSE) is compatible with the C-inner splicing specification element (CI-SSE) of the following integration strand.

The integration strands comprises a second and preferably a first DNA-sequence.

The first DNA-sequence comprises a N-inner splicing specification element (NI-SSE) and a N-inner polypeptide specification element (NI-PSE).

The N-inner splicing specification element (NI-SSE) is compatible with the C-inner splicing specification element of the following strand. The N-inner polypeptide specification element (NI-PSE) is compatible with the C-inner polypeptide specification element of the following strand. The N-inner polypeptide specification element is arranged adjacent and upstream of the N-inner splicing specification element (NI-SSE).

The second DNA-sequence is a 3'-end-part.

The 3'-end part comprises a C-inner splicing specification element. The C-inner polypeptide specification element (CI-PSE) is operably linked to the C-inner splicing specification element (CI-SSE). The C-inner splicing specification element (CI-SSE) is operably linked to the C-centered integration specification element (CC-ISE).

The 3'-centered integrase specific integration site is compatible with the 5'-centered integrase specific integration site. The C-inner splicing specification element (CI-SSE) compatible with the N-inner splicing specification element (NI-SSE) of the previous strand. The C-inner polypeptide specification element is compatible with the N-inner polypeptide specification element of the previous strand.

In this second set, all C-centered integration specification elements are compatible with all N-centered integration specification elements. Further, the combination of C-inner splicing specification element (CI-SSE) and C-inner polypeptide specification element of a given strand is only compatible with the combination of the N-inner polypeptide specification element and N-inner splicing specification elements (NI-SSE) of following strand in the desired sequence of strands to be integrated.

The previous strand is the host strand or the integration strand which is located adjacent to and upstream of the strand at hand while the following strand is the integration strand located adjacent to and downstream of the strand at hand in the desired sequence.

Preferably, the N-inner polypeptide specification element can be completed by a compatible C-inner polypeptide specification element to allow the expression of a marker.

Preferably, the second set comprises in addition a last integration strand. The last integration strand comprises a 3'-end part comprising a C-centered integration specification element, a C-inner splicing specification element (CI-SSE), a C-inner polypeptide specification element and a gene of interest to be integrated as the most downstream of all genes of interest to be integrated at the position at hand. The C-centered integration specification element of this last integration stand is compatible with the N-centered integration specification element of the previous integration strand. The C-centered integration specification element is adjacent to and upstream of the C-inner splicing specification element (CI-SSE). The C-inner splicing specification element is compatible with the N-inner splicing specification element (NI-SSE) of the previous integration strand. The C-inner splicing specification element is adjacent to and upstream of the C-inner polypeptide specification element. The C-inner polypeptide specification element is compatible with the N-inner polypeptide specification element of the previous strand such that their combination can express a marker.

The second set is preferably used to integrate multiple integration strands simultaneously at a desired integration site of a host strand using a single integrase. The successful integration in the desired sequence can be detected by the expression of all markers of the set.

Considering the sequence of integration strands, a previous strand is a strand arranged adjacent to and upstream of the strand at hand. A following strand is a strand arranged adjacent and downstream of the strand at hand.

A third set comprises two or more host strands and two or more integration strands.

Everyone of the host strands of the third set comprises a 5'-start part defining an integration site. The 5'-end part comprises a N-inner splicing specification element. The N-inner polypeptide specification element (NI-PSE) is operably linked to the N-inner splicing specification element (NI-SSE). The N-inner splicing specification element (NI-SSE) is operably linked to the N-centered integration specification element (NC-ISE).

Every one of the integration strands of the third set comprises a 3'-end part and genes of interest to be integrated in the host strand. The 3'-end part comprises a C-inner splicing specification element. The C-inner polypeptide specification element (CI-PSE) is operably linked to the C-inner splicing specification element (CI-SSE). The C-inner splicing specification element (CI-SSE) is operably linked to the C-centered integration specification element (CC-ISE).

In the third set, the combination of C-inner splicing specification element (CI-SSE) and C-inner polypeptide specification element of an integration strand is only compatible with the combination of N-inner polypeptide specification element and N-inner splicing specification element (NI-SSE) of the host strand in which it should be integrated.

The third set is preferably used to integrate different genes of interest in different host strands simultaneously. Successful integration can be confirmed by the detection of the expression of all markers of the set.

The possibility to integrate two different variants of a gene of interest at different locations allows to study homo-heterozygot phenotypes in an efficient manner.

A fourth set comprises a host strand and two or more integration strands to be integrated at a desired integration site in a desired sequence.

The host strand of the fourth set comprises 5'-start part defining the integration site. The 5'-end part comprises a N-inner splicing specification element. The N-inner polypeptide specification element (NI-PSE) is operably linked to the N-inner splicing specification element (NI-SSE). The N-inner splicing specification element (NI-SSE) is operably linked to the N-centered integration specification element (NC-ISE).

Every one of the integration strands is an integration strand comprising a first and a second DNA-sequence.

The first DNA-sequence comprises a N-inner splicing specification element (NI-SSE) and a N-inner polypeptide specification element (NI-PSE). The N-inner polypeptide specification element is arranged adjacent and upstream of the N-inner splicing specification element (NI-SSE).

The second DNA-sequence is a 3'-end part comprising a C-inner splicing specification element. The C-inner polypeptide specification element (CI-PSE) is operably linked to the C-inner splicing specification element (CI-SSE). The C-inner splicing specification element (Cl-SSE) is operably linked to the C-centered integration specification element (CC-ISE).

All C-inner polypeptide specification elements of any integration strand of the first embodiment of the fourth set are compatible with every one of the N-inner polypeptide specification element of the host strand or any integration strand of the first embodiment of the fourth set.

The previous strand is the host strand or the integration strand which is located adjacent to and upstream of the strand at hand while the following strand is the integration strand located adjacent to and downstream of the strand at hand in the desired sequence.

In a first embodiment of the fourth set, the C-inner splicing specification element of the second DNA-sequence comprises a first and a second 3'-inner splicing specification. The N-inner splicing specification element (NI-SSE) of the first DNA-sequence of a previous integration strand or of the host strand is in the first embodiment of the fourth set compatible to the first 3'-inner splicing specification of the second DNA sequence of the following integration strand.

In a second embodiment, the N-inner splicing specification element of the first DNA-sequence and the host strand comprises a first and a second 5'-inner splicing specification. The C-inner splicing specification element (CI-SSE) of the second DNA-sequence of a following integration strand is in the second embodiment of the fourth set compatible to the second 5'-inner splicing specification of the first DNA sequence of the previous integration strand.

The fourth set is preferably used to integrate different genes of interest simultaneously in a desired sequences and to determine faulty integration by detection of the expression of the marker.

The fifth set comprises a host strand and two or more integration strands to be integrated at a desired integration site in a desired sequence.

The host strand of the fifth set comprises the 5'-start part. The 5'-start part comprises an N-outer splicing specification element and an N-outer polypeptide specification element. The 5'-end part comprises a N-inner splicing specification element. The N-inner polypeptide specification element (NI-PSE) is operably linked to the N-inner splicing specification element (NI-SSE). The N-inner splicing specification element (NI-SSE) is operably linked to the N-centered integration specification element (NC-ISE).

The N-outer splicing specification element (NO-SSE) comprises a 5'- cis-intein encoding sequence. The N-outer splicing specification element (NO-SSE) is operably linked to the N-inner polypeptide specification element (NI-PSE). The N-outer splicing specification element (NO-SSE) is by itself splicing-incompetent. The N-outer splicing specification element (NO-SSE) can be rendered functional upon complementation by a compatible C-outer splicing specification element (CO-SSE).

The N-outer polypeptide specification element (NO-PSE) is operably linked to the N-outer splicing specification element (NO-SSE). The polypeptide encoded by the N-outer polypeptide specification element (NO-PSE) is by itself non-functional. The polypeptide encoded by the N-outer polypeptide specification element (NO-PSE) can be expressed from the expression cassette upon complementation by a compatible C-outer polypeptide specification element (CO-PSE).

Every one of the integration strands of the fifth set is an integration strand comprising a first and a second DNA-sequence.

The first DNA-sequence of the fifth set comprises from upstream to downstream and adjacent to each other, an N-outer polypeptide specification element, an N-outer splicing specification element, an N-inner polypeptide specification element (NI-PSE) and an N-inner splicing specification element (NI-SSE).

The second DNA-sequence of the fifth set is a 3'-end-part comprising the C-inner splicing specification element. The C-inner polypeptide specification element (CI-PSE) is operably linked to the C-inner splicing specification element (CI-SSE). The C-inner splicing specification element (CI-SSE) is operably linked to the C-centered integration specification element (CC-ISE).

The 3'end part of the second DNA-sequence of the fifth set comprises further a C-outer splicing specification element and a C-outer polypeptide specification element.

The C-outer splicing specification element (CO-SSE) comprises a 3'- cis-intein encoding sequence. The C-outer splicing specification element (CO-SSE) is operably linked to the C-inner polypeptide specification element (CI-PSE). The C-outer splicing specification element (CO-SSE) is by itself splicing-incompetent and can be rendered functional upon complementation by a compatible N-outer splicing specification element (NO-SSE).

The C-outer polypeptide specification element (CO-PSE) is operably linked to the C-outer splicing specification element (CO-SSE). The polypeptide encoded by the C-outer polypeptide specification element (CO-PSE) is by itself non-functional and can be expressed from the expression cassette upon complementation by a compatible N-outer polypeptide specification element (NO-PSE).

The N-inner splicing specification element (NI-SSE) of a previous strand of the fifth set is compatible to C-inner splicing specification element (CI-SSE) of the following integration strand of the fifth set.

The N-inner polypeptide specification element (NI-PSE) of a previous strand of the fifth set is only compatible with the C-inner polypeptide specification element (CI-PSE) of the following strand of the fifth set, resulting in the case of correct integration, in the expression of an inner marker.

The N-outer splicing specification element (NO-SSE) of a previous strand of the fifth set is not compatible to C-outer splicing specification element (CO-SSE) of the following integration strand of the fifth set.

The N- and C-outer splicing specification element (CO-SSE) of the same strand of the fifth set are compatible.

N-outer polypeptide specification element (NO-PSE) and the C-outer polypeptide specification element (CO-PSE) on the same strand of the fifth set are compatible resulting in the expression of an outer marker in the absence of an integration in the host strand.

The previous strand is the host strand or the integration strand which is located adjacent to and upstream of the strand at hand while the following strand is the integration strand located adjacent to and downstream of the strand at hand in the desired sequence.

Preferably, in the fifth set, the N-outer polypeptide specification element (NO-PSE) of a previous strand is not compatible with the C-outer polypeptide specification element (CO-PSE) of the following integration strand, resulting in the case of correct integration, in the absence of expression of an outer marker.

The fifth set is preferably used to integrate different genes of interest simultaneously in a desired sequence and to determine missing or faulty integration by detection of the expression of the outer marker and to determine successful integration by the detection of the expression of all inner markers occurring in the fifth set.

The sixth set comprises a host strand and two or more integration strands which are suitable to replace each other at the desired location of the host strand.

The host strand of the sixth set comprises a 5'-start part and a 3'-end part which are not compatible with each other. The integration strands of the sixth set are suitable to replace each other at the desired location of the host strand.

Every one of the integration strands of the sixth set comprises a 5'-start part and a 3'-end part and genes of interest arranged between the 5'-start part and the 3'-end part.

The different integration strands of the sixth set differ only in the genes of interest while all 5'-start parts and all a 3'-end parts are the same.

The 3'-end parts of the integration strands of the sixth set are compatible with the 5'-start part of the host strand of the sixth set such that in the case of successful integration a first marker can be expressed. The 5'-start part of the integration strands of the sixth set are compatible with the 3'-end part of the host strand of the sixth set such that in the case of successful integration a second marker can be expressed.

The incompatibility of the 5'-start part and a 3'-end part of the host strand is preferably realized by one of the following situations: In a first embodiment of the sixth set, the 5'-centred integrase specific integration site and the 3'-centred integrase specific integration site are orthogonal to each other. In a second embodiment of the sixth set the C-inner splicing specification element of the 3'-end part is not compatible with the N-inner splicing specification element of the 5'-start part. In a third embodiment of the sixth set, the 5'-centred integrase specific integration site and the 3'-centred integrase specific integration site are orthogonal to each other and the C-inner splicing specification element of the 3'-end part is not compatible with the N-inner splicing specification element of the 5'-start part.

The sixth set is preferably used in a cassette exchange process whereby a successful exchange can be detected by the expression of the first and the second marker.

A seventh set comprises one host strand and two or more integration strands.

The host strand of the seventh set comprises a 5'-start part and a 3'-end part which are not compatible with each other. The integration strands of the seventh set are suitable to replace each other at the desired location of the host strand.

Every one of the integration strands of the seventh set comprises a 5'-start part and a 3'-end part which are not compatible with each other, and genes of interest arranged between the two parts. The different integration strands of the seventh set differ only in the genes of interest while all 5'-start parts and all a 3'-end parts are the same.

The 3'-end parts of the integration strands of the seventh set are compatible with the 5'-start part of the host strand of the seventh set. The 5'-start part of the integration strands of the seventh set are compatible with the 3'-end part of the host strand of the seventh set.

The N-inner polypeptide specification element (NI-PSE) of the host strand, the C-inner polypeptide specification element (CI-PSE) of the integration strand, the N-inner polypeptide specification element (NI-PSE) of the integration strand and the C-inner polypeptide specification element (CI-PSE) of the host strand are combined the gene of interest of the seventh set. In the case of successful integration of an integration strand of the seventh set into the host strand of the seventh set, a desired version of the gene of interest is expressed.

In a particular preferred embodiment, the host strand comprises a N-outer polypeptide specification element (NO-PSE) arranged upstream of a N-outer splicing specification element (NO-SSE) which is arranged upstream and operably linked to the N-inner polypeptide specification element (NI-PSE) of the host strand. The host strand of this embodiment comprises further a C-outer splicing specification element (CO-SSE) which is arranged downstream and operably linked to the C-inner polypeptide specification element (CI-PSE) of the host strand which is arranged upstream of a C-outer polypeptide specification element (CO-PSE). The N- and C-outer splicing specification element (NO-SSE, CO-SSE) of this host strand are compatible with each other. The N- and C-outer polypeptide specification element (NO-PSE, CO-PSE) of this host strand are compatible with each other. In the case of successful integration of an integration strand of the seventh set into the host strand of this embodiment of a seventh set, the outer marker is expressed.

Preferably, the seventh set is used in a cassette exchange process whereby only a part of the gene of interest is exchanged and whereby a successful exchange can be detected by the expression of the outer marker.

The eighth set comprises a 5'-start part, a first DNA sequence comprising a N-inner splicing specification element (NI-SSE) and a N-inner polypeptide specification element (NI-PSE) and two 3'-end parts. The 5'-start part and one of the two 3'end parts are part of a first expression cassette. The first DNA sequence and the other one of the two 3'end parts are part of a second expression cassette. The N-inner polypeptide specification element (NI-PSE) of the 5'-start part of the first expression cassette and the C-inner polypeptide specification element (CI-PSE) of the 3'-end part of the first expression cassette can be combined to result in a first non-functional half of a marker. The N-inner polypeptide specification element (NI-PSE) of the first DNA sequence of the second expression cassette and the C-inner polypeptide specification element (CI-PSE) of the 3'-end part of the second expression cassette can be combined to result in a second non-functional half of a marker.

The 5'-start part, the first DNA sequence and the two 3-end parts are arranged in any one of the following ways:
In a first embodiment, the first DNA sequence is part of a second 5'-start part. Both 5'-start parts are arranged at different locations on the same host strand and every one of the 3'end parts is arranged on a different integration strand.

In a second embodiment, the first DNA sequence is part of a second 5'-start part. The 5'-start parts are arranged on the different host strands and every one of the 3'end parts is arranged on a different integration strand.

In a third embodiment, the 5'-start part is arranged on a host strand. Every one of the 3'-end parts is arranged on a different integration strand. The first DNA sequence is arranged on the integration strand which carries the 3'-end part of the expression cassette to which the 5'-start part belongs.

In one variant of any one of the embodiments of the eighth set, the first non-functional half of the marker comprises a 5'-trans-intein encoding sequence and the second non-functional half of the marker comprises a 3'-trans-intein encoding sequence. In this way the marker can be expressed by trans-splicing.

In another variant of any one of the embodiments of the eighth set, the marker is a split marker which can be expressed functionally even if its encoding sequences are separated. In this case the marker can be expressed without the need for further splicing processes once the two, separately, non-functional halves of the marker are created.

Thereby, upon successful integration of both integration strands of the eighth set into the host strand or the host strands of the eighth set, the first and the second non-functional half of a marker can be expressed to result in a functional marker.

The eighth set is preferably used to integrate two genes of interest simultaneously either in a desired sequence at a desired location or at two desired locations on one or two host strands, whereby a successful integration can be detected by the expression of one single marker.

A ninth set comprises one host strand and one integration strand to be integrated.

The host strand of the ninth set comprises a 5'-start part. The 5'-start part comprises in addition to the N-centered integration specification element (NC-ISE), the N-inner polypeptide specification element (NI-PSE), and the N-inner splicing specification element (NI-SSE), a N-outer splicing specification element and a N-outer polypeptide specification element (NO-PSE). The 5'-start part defines the desired integration location.

The N-outer splicing specification element (NO-SSE) comprises a 5'- cis-intein encoding sequence. The N-outer splicing specification element (NO-SSE) is operably linked to the N-inner polypeptide specification element (NI-PSE). The N-outer splicing specification element (NO-SSE) is by itself splicing-incompetent and can be rendered functional upon complementation by a compatible C-outer splicing specification element (CO-SSE).

The N-outer polypeptide specification element (NO-PSE) is operably linked to the N-outer splicing specification element (NO-SSE). The polypeptide encoded by the N-outer polypeptide specification element (NO-PSE) is by itself non-functional and can be expressed from the expression cassette upon complementation by a compatible C-outer polypeptide specification element (CO-PSE).

The integration strand of the ninth set comprises a 3'-end part. The 3'-end part comprises in addition to the C-centered integration specification element (CC-ISE), the C-inner splicing specification element (CI-SSE) and the C-inner polypeptide specification element (CI-PSE), a C-outer splicing specification element (CO-SSE) and a C-outer polypeptide specification element (CO-PSE).

The C-outer splicing specification element (CO-SSE) comprises a 3'- cis-intein encoding sequence. The C-outer splicing specification element (CO-SSE) is operably linked to the C-inner polypeptide specification element (CI-PSE). The C-outer splicing specification element (CO-SSE) is by itself splicing-incompetent and can be rendered functional upon complementation by a compatible N-outer splicing specification element (NO-SSE).

The C-outer polypeptide specification element (CO-PSE) is operably linked to the C-outer splicing specification element (CO-SSE). The polypeptide encoded by the C-outer polypeptide specification element (CO-PSE) is by itself non-functional and can be expressed from the expression cassette upon complementation by a compatible N-outer polypeptide specification element (NO-PSE).

The N-centered integration specification element (NC-ISE) of the host strand of the ninth set is compatible to the C-centered integration specification element (CC-ISE) of the integration strand of the ninth set, facilitating the desired integration.

The N-inner splicing specification element (NI-SSE) of the host strand of the ninth set is compatible to the C-inner splicing specification element (CI-SSE) of the integration strand of the ninth set.

The N-inner polypeptide specification element (NI-PSE) of the host strand of the ninth set is compatible with the C-inner polypeptide specification element (CI-PSE) of the integration strand of the ninth set, resulting in the case of correct integration, in the expression of an inner marker.

The N-outer splicing specification element (NO-SSE) of the host strand of the ninth set is compatible to C-outer splicing specification element (CO-SSE) of the integration strand of the ninth set.

The N-outer polypeptide specification element (NO-PSE) of the host strand of the ninth set is compatible with the C-outer polypeptide specification element (CO-PSE) of the integration strand of the ninth set, resulting in the case of correct integration, in the expression of an outer marker.

Preferably, the ninth set is used as logical OR-gate, where a first trigger condition causing the N-inner splicing specification element and the C-inner splicing specification element to splice and a second trigger condition causing the N-outer splicing specification element and the C-outer splicing specification element to splice, are the input variables. The output of the OR-gate is the expression of any one of the inner or the outer marker, such that if either the first or the second or both trigger conditions are met, either the inner or the outer or both markers are expressed. Furthermore, the splicing-dependent expression of the expression cassette can be controlled by two different splicing reactions.

Preferably, the N- or the C-centered integration specification element comprises a recombinase recognition site which reacts with one of the following integrases:
Cre, Dre, Flp, lambda integrase, gamma-delta resolvase, Tn3 resolvase, Sin resolvase, Gin invertase, Hin invertase, Tn5044 resolvase, IS607 transportase, Bxb1, wBeta, BL3, phiR4, A118, TG1, MR11, phi370, SPBc, TP901-1, phiRV, FC1, K38, phiBT1, phiC31.

Particularly preferably, a Tyr recombinase is used in all of the above described embodiments where an integration strand comprises a first DNA sequence which is not part of a 5'-start part or a second DNA-sequences which is not part of a 3'-end part. A preferred Tyr recombinase is one of the following: Cre, Dr, FLP, KD, B2, B3.

Preferably, the N- or the C-centered integration specification elements comprises a recombinase recognition site according to one of the following orthogonal wild types or their mutants: attB, attP, FRT, IoxP.

Preferably, the N- or the C- inner splicing specification element comprises the phase-specific 5'- intron sequence or the phase-specific 3'- intron sequence. In this embodiment, the phase-specific 5'- intron sequence or the phase-specific 3'- intron sequence is a 5'- or 3-' sequence of an intron of one of the following wild types or their mutants or artificially variants:
1st intron of TUBA1A, 1st, 2nd, 5th, 6th, 7th or 10th intron of MAP11, 1st intron of TUBA1B, 1st intron of TUBA1C, 2nd, 5th, 14th, 15th, 17th intron of PPP1R12C, 2nd intron of TUBB2A, 2nd intron of TUBB2B, 2nd intron of TUBB4A, 2nd intron of TUBB4B, 2nd intron of TUBB8, 2nd, 10th or 16th intron of TYR03, 3rd intron of TUBB1, 7th or 9th intron of TUBG1, 7th, 9th of 10th intron TUBG2.

Preferably, at least one of the N- or C- inner or outer splicing specification element" (NI-SSE. CI-SSE, NO-SSE, CO-SSE) comprises the 5'- or 3'- cis-intein encoding sequence of a fragment of a wild type, artificial or mutant cis-intein, preferably SspDnaB, NpuDnaE.

Preferably, at least one of the polypeptide specification elements (NI-PSE. CI-PSE, NO-PSE, CO-PSE) comprises a fragment of a DNA sequence encoding at least one marker.

The N-inner polypeptide specification element, the N-outer polypeptide specification element, the C-inner polypeptide specification element and the C-outer polypeptide specification element are polypeptide specification elements.

Preferably, at least one of the polypeptide specification elements (NI-PSE. CI-PSE, NO-PSE, CO-PSE) comprises a DNA sequence encoding a split-protein. The split-protein of this embodiment is partitioned into two or more split-intein fragments reconstituted by protein trans-splicing. The split-protein of this embodiment comprises at least one marker.

Preferably, at least one of the polypeptide specification elements (NI-PSE. CI-PSE, NO-PSE, CO-PSE) comprises a DNA sequence encoding a fused-protein. The fused-protein according to this embodiment comprises two or more polypeptide fragments which comprise at least one marker.

Preferably, the marker is a 2A-peptide of the following wild types or their mutants:
- P2A (Porcine teschovirus 1), T2A (Thosea asigna virus), E2A (Equine rhinitis A Virus), F2A (Foot and mouth disease virus)
- a wild type, artificial or mutant IRES element
- a wild type, artificial or mutant a gene of interest, which is capable to produce a readout signal without an additional selection marker
- a wild type, artificial or mutant toxic protein, preferably a diphteria toxin or thymidine kinase
- a wild type, artificial or mutant light-sensitive protein
- a wild type, artificial or mutant bioluminescent or fluorescent marker protein
- a wild type, artificial or mutant antibiotic resistance marker
- a wild type, artificial or mutant ligand-responsive repressor protein, preferably tetracycline or cumate
- a wild type, artificial or mutant receptor, transporter, ion channel, stretch-activated channel, water channel, calcium or voltage sensor protein
- a wild type, artificial or mutant nuclease
- a wild type, artificial or mutant molecular tag
- wild type, artificial or mutant integrase or transposon
- a wild type, artificial or mutant kinase or ubiquitinligase.
- a wild type, artificial or mutant transcription factor
- a wild type, artificial or mutant RNA-binding protein, preferably FUS

Other advantageous embodiments and combinations of features come out from the detailed description below and the entirety of the claims.

### Brief description of the drawings

The drawings used to explain the embodiments show:
- Fig. 1: Host and integration strand of a first set as well as host strand with integrated integration strand in the application of the invention to high precision integration and off-target prevention. After successful integration this host strand can be used as logical AND-gate.
- Fig. 2: Host and three different integration strands of a second set as well as host strand with all integration strands integrated in a desired sequence and in a undesired sequence in the application of the invention to multiplex integration at single site.
- Fig. 3: Two host and two different integration strands of a third set as well as both host strands with both integration strands integrated in a desired position in the application of the invention to multiplex integration at multiple sites.
- Fig. 4: A host and two different integration strands of a fourth set as well as the host strand with both integration strands integrated in a desired sequence and in a undesired sequence in the application of the invention to detect correct integration by a single marker.
- Fig. 5: A host and two different integration strands of a fifth set as well as the host strand with all integration strands integrated in a desired sequence in the application of the invention to detect correct integration by a combination of markers.
- Fig. 6: A host and an integration strand as well as the host strand of a sixth set with the integration strand integrated in at a desired position in the application of the invention to a cassette exchange procedure.
- Fig. 7: A host and an integration strand as well as the host strand of a seventh set with the integration strand integrated in at a desired position in the application of the invention to mutagenesis.
- Fig. 8: A host and two different integration strands as well as the host strand of an eighth set with both integration strands integrated in a desired sequence in the application of the invention to detect correct integration by a single split-split marker.
- Fig. 9: A host strand with an integrated integration strand illustrating the use as logical OR gate.

In the figures, the same components are given the same reference symbols.

### Preferred embodiments

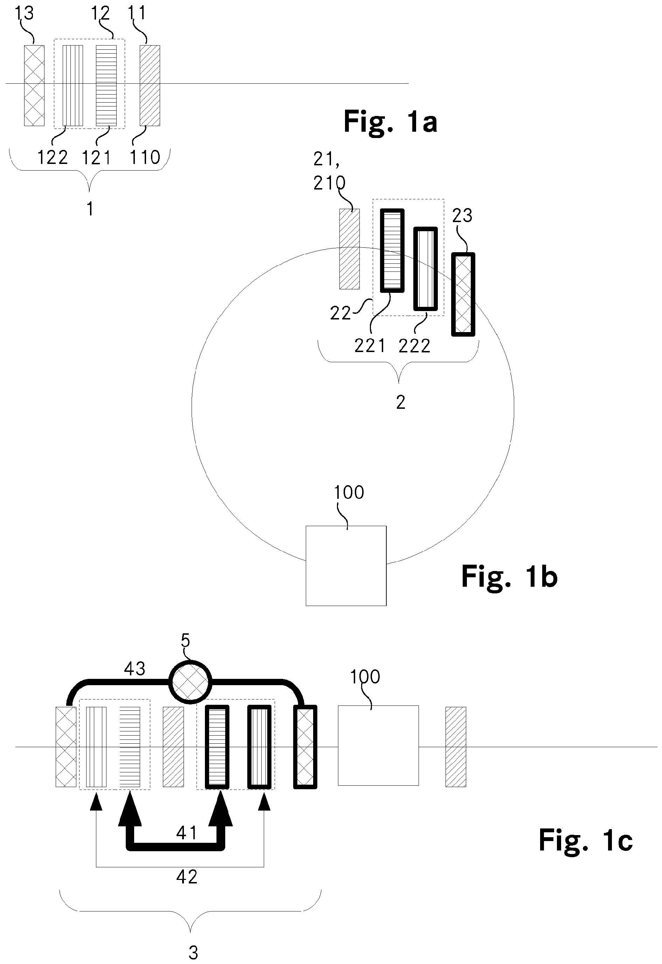

Figure 1a shows a host strand. Figure 1b shows integration strand and Figure 1c the host strand with the correctly integrated integration strand. The host strand according to figure 1a and the integration strand according to Figure 1b form a first set according to the invention.

The first set as shows can be used for high precision integration and off-target prevention as well as as logical AND gate.

The host strand comprises a 5'-start part (1). The 5'-start part (1) comprises, from upstream to downstream the following elements: a N-inner polypeptide specification element (13), a N-inner splicing specification element (12) and N-centered integration specification element (11). The N-inner splicing specification element (12) comprises, from upstream to downstream, the 5'- cis-intein encoding sequence (122) and the phase-specific 5'- intron sequence (121). The N-centered integration specification element (11) comprises a 5'-centered integrase specific integration site (110).

The integration strand, shown in Figure 1b, is a plasmid. It comprises the 3'-end part (2) of the expression cassette. The 3'-end part (2) comprises, from upstream to downstream, the following elements: a C-centered integration specification element (21), a C-inner splicing specification element (22) and a C-inner polypeptide specification element (23) and a gene of interest 100. The C-inner splicing specification element (22) comprises, from upstream to downstream, the phase-specific 3'- intron sequence (221) and the 3'-cis-intein encoding sequence (222). The C-centered integration specification element (21) comprises a 3'-centered integrase specific integration site (210).

In this first set, comprising the host strand of Figure 1a and the integration strand of Figure 1b, the 5-start part (1) and the 3'-end part (2) are compatible with each other.

Figure 1c shows the host strand after successful integration of the integration stand. The 5'-start part (1) is arranged upstream and adjacent to the 3'-end part (2) such that the expression cassette (3) is completed. As the phase-specific 5' and 3'-intron sequences (121, 221) are compatible with each other and arranged in sufficient proximity from each other, intron splicing (41) is possible. Successful intron splicing (41) brings the compatible 5' and 3'- cis-intein encoding sequences (122, 222) on the mRNA sufficiently close to each other that intein splicing (42) is possible. The result of the intein splicing (42) is the expressed (43) polypeptide specification element which is a polypeptide with a known and measurable property, for example a fluorescence. This fluorescent protein is used as a marker (5) to indicate a successful integration.

An example of such a 5'-start part and a 3'- end part is given in the sequence listings SEQ ID No. 1 and SEQ ID No. 2.

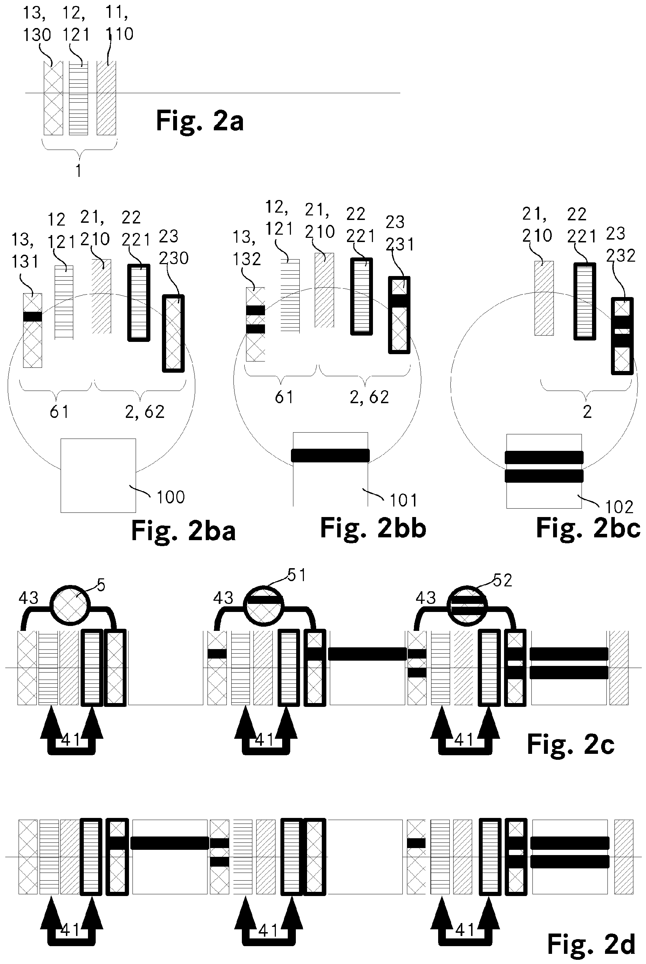

Figure 2a shows a host strand. Figures 2ba, 2bb and 2bc show three different integration strands. The strands shown in Figures 2a, 2ba, 2bb and 2bc are part of a second set.

The host strand comprises a 5'-start part (1). The 5'-start part (1) comprises the N-inner polypeptide specification element (13), the N-inner splicing specification element (12) and the N-centered integration specification element (11).

The N-inner polypeptide specification element (13, 130) is compatible with the C-inner polypeptide specification element (23, 230) of the following integration strand, which is shown in Figure 2ba. The N-inner polypeptide specification element (13, 130) is arranged adjacent and upstream of the N-inner splicing specification element (12). The N-inner splicing specification element (12) is compatible with the C-inner splicing specification element (22) of the following integration strand, which is shown in Figure 2ba. The N-inner splicing specification element (12) is arranged adjacent to and upstream of the N-centered integration specification element (11). The N-centered integration specification element (11) comprises a 5'-centered integrase specific integration site (110).

The integration strands, shown in Figures 2ba, 2bb and 2bc, comprise a first (61) and a second DNA-sequence (62).

The second DNA-sequence (62) is a 3'-end part (2). The 3'-end part (2) consists of the C-centered integration specification element (21) arranged upstream and adjacent to the C-inner splicing specification element (22) which in turn is arranged upstream and adjacent to the C-inner polypeptide specification element (23). Downstream of the 3'-end part (2), there is a gene of interest (100, 101, 102) arranged on the integration strand. In the integration strands shown in Figures 2ba and 2bb, there is downstream of the gene of interest (100, 101) a first DNA-sequence (61). The C-centered integration specification element (21) comprises a 3'-centered integrase specific integration site (210).

The first DNA-sequence (61) comprises, from upstream to downstream and adjacent to each other the N-inner polypeptide specification element (13) and the N-inner splicing specification element (12).

The C-centered integration specification element (21) of the integration stands shown in Figures 2ba, 2bb and 2bc is compatible with the N-centered integration specification element (11) of the host strand shown in Figure 2a, as the integrase to which the 5'-centered integrase specific integration site (110) and the 3'-centered integrase specific integration site (210) are specific to is the same. The C-inner splicing specification element (22) is compatible with the N-inner splicing specification element (12) of the previous strand. The C-inner polypeptide specification element (23, 230, 231, 232) is compatible with the N-inner polypeptide specification element (13, 130, 131, 132) such that they can express a marker (5, 51, 52) when they are combined.

The previous strand of the integration strand shown in Figure 2ba is the host strand as shown in Figure 2a. The previous strand of the integration strand shown in Figure 2bb is the integration strand shown in Figure 2ba. The previous strand of the integration strand shown in Figure 2bc is the integration strand shown in Figure 2bb. Consequently, the C-inner polypeptide specification element (230) of the integration strand shown in Figure 2ba is compatible with the N-inner polypeptide specification element (130) of the host strand shown in Figure 2a and together they can express the first marker (5). The C-inner polypeptide specification element (231) of the integration strand shown in Figure 2bb is compatible with the N-inner polypeptide specification element (131) of the integration strand shown in Figure 2ba and together they can express the second marker (51). The C-inner polypeptide specification element (232) of the integration strand shown in Figure 2bc is compatible with the N-inner polypeptide specification element (132) of the integration strand shown in Figure 2bb and together they can express the third marker (52).

The N-inner splicing specification element (12) of the integration stands shown in Figures 2ba and 2bb is compatible with the C-inner splicing specification element (22) of the following strands.

In the second set as shown in Figures 2a and 2ba, 2bb, 2bc, all N-inner splicing specification elements (12) are the same and all C-inner splicing specification elements (22) are the same and therefore, every N-inner splicing specification element (12) in the depicted second set is compatible with every C-inner splicing specification element (22).

In this second set, all C-centered integration specification elements (21) are compatible with all N-centered integration specification elements (11).

However, the combination of the C-inner splicing specification element (22) and C-inner polypeptide specification element (23) of a given strand is only compatible with the combination of N-inner polypeptide specification element (13) and N-inner splicing specification element (12) of following strand in the desired sequence of strands to be integrated. In the case shown in Figures 2a and 2ba, 2bb and 2bc, this requirement is fulfilled by the C-inner polypeptide specification elements (23) and the N-inner polypeptide specification elements (13). The second set as shown in Figures 2a, 2ba, 2bb and 2bc, the desired sequence is that the integration strand according to Figure 2ba is most upstream, followed by the integration strand according to Figure 2bb further downstream, followed by the integration strand according to Figure 2bc as the most downstream strand.

The second set shown in Figures 2a, 2ba, 2bb and 2bc comprises a last integration strand which is shown in Figure 2bc. The last integration strand comprises a C-centered integration specification element (21), a C-inner splicing specification element (22) and C-inner polypeptide specification element (23) and a gene of interest (102) to be integrated as the most downstream of all genes of interest (100, 101, 102). The C-centered integration specification element (21) of the last integration stand shown in Figure 2bc is compatible with the N-centered integration specification element (21) of the previous integration strand, shown in Figure 2bb. The C-centered integration specification element (21) is adjacent to and upstream of the C-inner splicing specification element (22). The C-inner splicing specification element (22) is compatible with the N-inner splicing specification element (12) of the previous integration strand, which is shown in Figure 2bb. The C-inner splicing specification element (22) is adjacent to and upstream of the C-inner polypeptide specification element (23) of the third marker (52). The C-inner polypeptide specification element (23) is compatible with the N-inner polypeptide specification element of the previous integration strand, which is shown in Figure 2bb.

In the depicted embodiment, the N-inner splicing specification element (12) is in the host strand shown in Figure 2a and the integration strands shown in Figures 2ba, 2bb and 2bc, a phase-specific 5'- intron sequence (121). The C-inner splicing specification element (22) is in the integration strands shown in Figures 2ba, 2bb and 2bc, the compatible phase-specific 3'- intron sequence (221).

Figure 2c shows the host strand with all integration strands integrated in the desired sequence. There are three expression cassettes realized, each of them comprising a nested arrangement of the integration specification element, the splicing specification element and the polypeptide specification element. The genes of interest are arranged between the expression cassettes. Intron splicing (41) combines in every one of the cassettes compatible N-inner polypeptide specification elements and C-inner polypeptide specification elements. The first cassette results therefore in the expression (43) of the first marker (5), indicating that the first gene of interest 100 is integrated downstream of the 5'-start part (1). The second cassette results in the expression (43) of the second marker (51), indicating that the second gene of interest 101 is integrated downstream of the first gene of interest (100). The third cassette results in the expression (43) of the third marker (52), indicating that the third gene of interest 102 is integrated downstream of the second gene of interest (101). Consequently, the presence of the first marker (5), the second marker (51) and the third marker (52) at the same time indicate the successful integration of all genes of interest in the desired sequence.

Figure 2d shows the host strand with all integration strands integrated but in an unwanted sequence. While the intron splicing (41) will take place as the integration specification elements, the splicing specification elements, are compatible in the depicted second set, none of the markers is expressed as the N-inner polypeptide specification elements and C-inner polypeptide specification elements which are combined by the intron splicing are incompatible.

Therefore, a second set according to the invention allows the multiplex integration of multiple integration strands in a desired sequence at single site of the host strand: All integrations which were not successful can be eliminated by screening for the presences of the applicable markers.

The second set can also be realised by using a 5'- cis-intein encoding sequence as N-inner splicing specification element (12) and a 3'- cis-intein encoding sequence as C-inner splicing specification element (22). In this embodiment, successful integration allows the expression of all markers by intein splicing instead of intron splicing. The second set can further use N-inner splicing specification elements (12) which are a combination of 5'-cis-intein encoding sequences and phase-specific 5'- intron sequences if the C-inner splicing specification elements (22) are compatible combinations of 3'- cis-intein encoding sequences and phase-specific 3'- intron sequences.

An example of such a 5'-start part of a host strand and a 3'- end parts as well as first DNA-sequences (61) suitable to form a second set is given in the sequence listing SEQ ID No. 3, SEQ ID No. 4 and SEQ ID No. 5

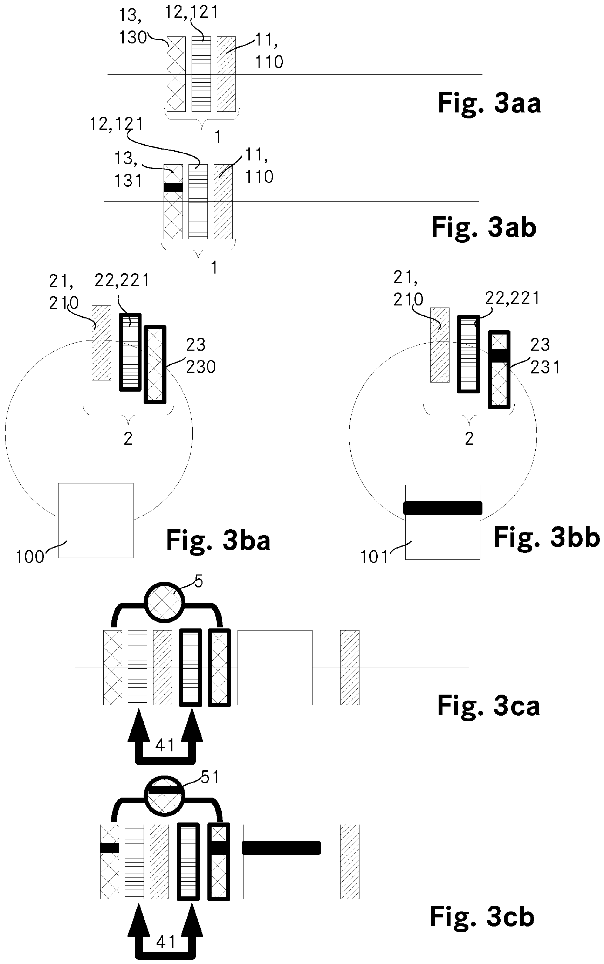

Figure 3aa shows a first host strand. Figure 3ab shows a second host strand. Figure 3ba shows a first integration strand to be integrated in the first host strand and Figure 3bb shows a second integration strand to be integrated in the second host strand. The strands depicted in Figures 3aa, 3ab, 3ba and 3bb form a third set. The third set allows a multiplex integration of multiple integration strands at multiple sites of one or more host strands.

The host strands of the third set, shown in Figures 3aa and 3ab comprises a 5'-start part (1) defining the desired integration sites. The 5'-start parts (1) comprise, upstream to downstream and adjacent to each other, an N-inner polypeptide specification element (13), an N-inner splicing specification element (12) and an N-centered integration specification element (11). While the N-inner splicing specification element (12) and an N-centered integration specification element (11) are the same on both host strands shown in Figures 3aa and 3ab, the host strands differ at least in the N-inner polypeptide specification elements (13): The N-inner polypeptide specification element (130) of the first host strand depicted in Figure 3aa can be completed to express a first marker (5). The N-inner polypeptide specification element (131) of the second host strand depicted in Figure 3ab can be completed to express a second marker (51). The N-centered integration specification element (11) comprises a 5'-centered integrase specific integration site (110).

Every one of the integration strands of the third set comprises a 3'-end part (2) and genes of interest (100,101) to be integrated in the host strand. The 3'-end parts (2) comprise upstream to downstream and adjacent to each other, a C-centered integration specification element (21), a C-inner splicing specification element (22) and a C-inner polypeptide specification element (23). While the C-inner splicing specification element (22) and an C-centered integration specification element (21) are the same on both integration strands shown in Figures 3ba and 3bb, the integration strands differ at least in the C-inner polypeptide specification elements (23) and the genes of interest (100, 101): The C-inner polypeptide specification element (230) of the first integration strand depicted in Figure 3ba can be completed to express a first marker (5). The C-inner polypeptide specification element (231) of the second integration strand depicted in Figure 3bb can be completed to express a second marker (51). The C-centered integration specification element (21) comprises a 3'-centered integrase specific integration site (210).

Figures 3ca shows the first host strand as depicted in Figure 3aa with the first integration strand as depicted in Figure 3ba integrated at the desired position. In this case the 5'-start part (1) and the 3'-end part (2) form an expressible and functional expression cassette. Upon intron splicing (41), the two compatible inner polypeptide specification elements are combined and the first marker (5) is expressed, indicating the successful integration.

Figures 3cb shows the second host strand as depicted in Figure 3ab with the second integration strand as depicted in Figure 3bb integrated at the desired position. In this case the 5'-start part (1) and the 3'-end part (2) form an expressible and functional expression cassette. Upon intron splicing (41), the two compatible inner polypeptide specification elements are combined and the second marker (5) is expressed, indicating the successful integration.

The third set is preferably used to integrate different genes of interest (100, 101) in different host strands simultaneously. Successful integration can be confirmed by the detection of the expression of all markers (5, 51) of the set. The possibility to integrate two different genes of interest at different locations allows to study homo-heterozygot phenotypes in an efficient manner.

The host strands of the third set as shown in Figure 3aa, 3ab use a phase-specific 5'-intron sequence (121) as N-inner splicing specification element (12). The integration strands of the third set as shown in Figure 3ba, 3bb use a phase-specific 3'- intron sequence (221) as C-inner splicing specification element (12).

However, the third set can also be realised by using a 5'- cis-intein encoding sequence as N-inner splicing specification element (12) and a 3'- cis-intein encoding sequence as C-inner splicing specification element (22). In this embodiment, successful integration allows the expression of all markers by intein splicing instead of intron splicing. The second set can further use N-inner splicing specification elements (12) which are a combination of 5'- cis-intein encoding sequences and phase-specific 5'-intron sequences if the C-inner splicing specification elements (22) are compatible combinations of 3'- cis-intein encoding sequences and phase-specific 3'- intron sequences.

Further, Figures 3aa and 3ab depict the two different 5'-start parts (1) to be arranged on two different host strands. However, more than two different 5'-start parts (1) can be arranged the same way on more than two different host strands to increase the number of orthogonally specified integration sites and therefore multiplex applicability. In another embodiment, two or more different 5'-start parts (1) are arranged on the same host strand, allowing a controlled multiplex integration of different genes of interest at different positions on this host strand.

An example of such 5'-start parts of host strands and a 3'-end part suitable to form a third set is given in the sequence listing SEQ ID No. 6, SEQ ID No. 7 and SEQ ID No. 8.

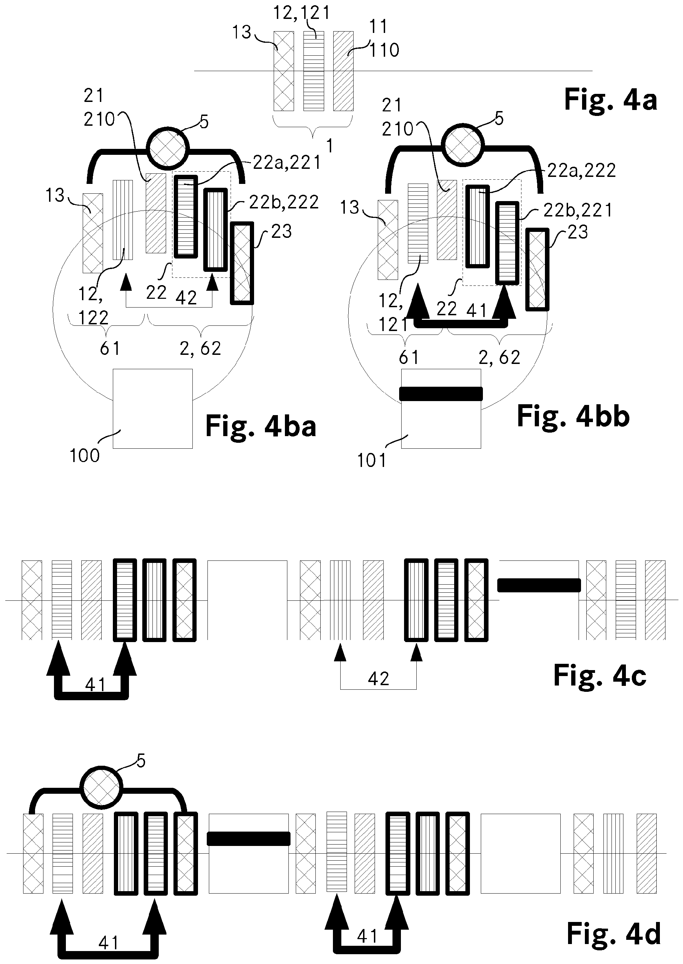

Figure 4a shows a host strand of a fourth set. Figures 4ba and 4bb show two different integration strands of the fourth set. Figure 4c shows the integration stands according to Figures 4ba and 4bb correctly integrated in the host strand of Figure 4a. Figure 4d shows a wrong integration of the integration strands of Figure 4ba and 4bb as they are integrated in an unwanted sequence. Expression of the marker (5) indicates a wrong or missing integration in the case of a fourth set.

The host strand shown in Figure 4a of the fourth set comprises a 5'-start part (1) defining the integration site. The 5'-start part (1) comprises, upstream to downstream and adjacent to each other, an N-inner polypeptide specification element (13), an N-inner splicing specification element (12) and an N-centered integration specification element (11). The N-centered integration specification element (11) comprises a 5'-centered integrase specific integration site (110).

Every one of the integration strands, shown in Figures 4ba and 4bb, comprises a first (61) and a second DNA-sequence (62).

The second DNA-sequence (62) is a 3'-end part (2). The 3'-end part (2) consists of the C-centered integration specification element (21) arranged upstream and adjacent to the C-inner splicing specification element (22) which in turn is arranged upstream and adjacent to the C-inner polypeptide specification element (23). Downstream of the 3-end part (2), there is a gene of interest (100, 101) arranged on the integration strand. In the integration strands shown in Figures 4ba and 4bb, there is downstream of the gene of interest (100, 101) a first DNA-sequence (61). The C-centered integration specification element (21) comprises a 3'-centered integrase specific integration site (210).

The first DNA-sequence (61) comprises, from upstream to downstream and adjacent to each other the N-inner polypeptide specification element (13) and the N-inner splicing specification element (12).

The C-inner splicing specification element (22) of the second DNA-sequence comprises a first (22a) and a second 3'-inner splicing specification (22b). The N-inner splicing specification element (NI-SSE) of the first DNA-sequence (61) of a previous integration strand or of the host strand is in the fourth set compatible to the first 3'-inner splicing specification (22a) of the second DNA sequence (62) of the following integration strand.

The C-inner polypeptide specification element (23) of any integration strand of the fourth set is compatible with every N-inner polypeptide specification element (13) of the host strand and any integration strand of the fourth set.

In the fourth set shown in Figures 4a, 4ba and 4bb, the N-inner splicing specification element (12) of the host strand and the second integration strand as shown in Figure 4bb is a phase-specific 5'-intron sequence (121). The N-inner splicing specification element (12) of the first integration strand as shown in Figure 4ba is a 5'-cis-intein encoding sequence (122). The C-inner splicing specification element (22) of the first and the second integration strand as shown in Figures 4ba and 4bb is a combination of a phase-specific 3'- intron sequence (221) which is compatible with the phase-specific 5'- intron sequence (121) occurring in the fourth set and a 3'- cis-intein encoding sequence (222) which is compatible with the 5'- cis-intein encoding sequence (122) occurring in the fourth set. In Figure 4ba the phase-specific 3'-intron sequence (221) is upstream of the 3'- cis-intein encoding sequence (222). In Figure 4bb the 3'- cis-intein encoding sequence (222) is upstream of the phase-specific 3'- intron sequence (221).

If an intein splicing (42) takes place at the unintegrated first integration strand according to Figure 4ba, the 5'- cis-intein encoding sequence (122) of the first DNA sequence (61) and the 3'- cis-intein encoding sequence (222) of the second DNA sequence (62) are involved in the splicing, combining thereby the N-inner polypeptide specification element with the C-inner polypeptide specification element such that a marker (5) is expressed.

Similarly, if an intron splicing (41) takes place at the unintegrated second integration strand according to Figure 4bb, the phase-specific 5'- intron sequence (121) of the first DNA sequence (61) and the phase-specific 3'- intron sequence (221) of the second DNA sequence (62) are involved in the splicing, combining thereby the N-inner polypeptide specification element with the C-inner polypeptide specification element such that a marker (5) is expressed.

If the first integration strand according to Figure 4ba is integrated downstream and adjacent to the 5'-start part (1) in the host strand according to Figure 4a, a first expression cassette is completed and if the second integration strand according to Figure 4bb is integrated downstream and adjacent to the first DNA sequence (61) of the first integration strand according to Figure 4ba, a second expression cassette is completed.

The first expression cassette comprises a phase-specific 5'- intron sequence (121), a phase-specific 3'- intron sequence (221) and a 3'- cis-intein encoding sequence (222). Consequently, only intron splicing (41) is possible. After the intron splicing, the 3'- cis-intein encoding sequence (222) is arranged between the N-inner polypeptide specification element and C-inner polypeptide specification element and stops the expression of the marker.

The second expression cassette comprises a 5'- cis-intein encoding sequence (122), a phase-specific 3'- intron sequence (221) and a 3'- cis-intein encoding sequence (222). Consequently, only intein splicing (42) is possible. After the intein splicing, the phase-specific 3'- intron sequence (221) is arranged between the N-inner polypeptide specification element and C-inner polypeptide specification element and stops the expression of the marker.

Therefore, successful integration in the desired sequence results in the lack of expressed markers (5).

Figure 4d shows the situation of an integration in the undesired sequence. As in this case, the 3'- cis-intein encoding sequence (222) is arranged upstream of the phase-specific 3'-intron sequence (221), the intron splicing (41) eliminates the 3'- cis-intein encoding sequence (222) as well as the integration specification element (21). Therefore the N-inner polypeptide specification element is combined with the C-inner polypeptide specification element and the marker (5) is expressed, indicating an undesired integration.

Therefore, the fourth set can used to integrate different genes of interest in a desired sequences and to determine faulty integration by detection of the expression of a single marker.

In an analogue but not shown embodiment the N-inner splicing specification element (12) comprises two splicing specifications, such as a phase-specific 5'- intron sequence and a 5'-cis-intein encoding sequence. In such an embodiment, the C-inner splicing specification element (22) comprises only one splicing specification.The splicing logic of this embodiment is analogue to the one shown in Figures 4c and 4d.

An example of a 5'-start part (1) and a 3'-end parts (2) as well as first DNA sequences (61) suitable to form a fourth set are given in the sequence listing SEQ ID No. 10, SEQ ID No. 11 and SEQ ID No. 12.

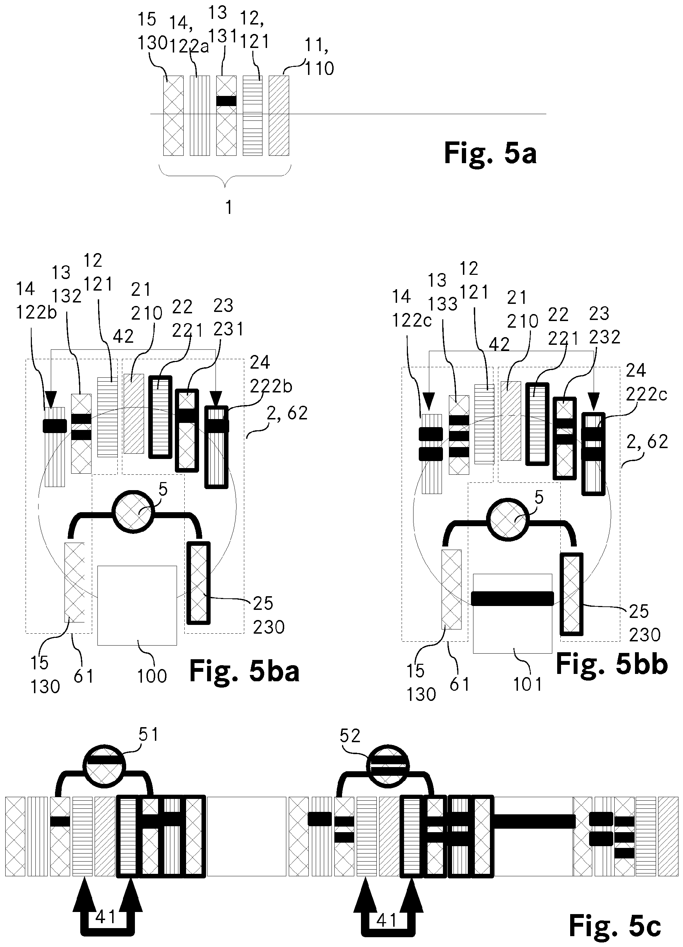

Figure 5a shows a host strand of a fifth set. Figures 5ba and 5bb show two integration strands of a fifth set. Figure 5c shows the integration strands according to Figures 5ba and 5bb integrated in the host strand according to Figure 5a.

The host strand of the fifth set shown in Figure 5a comprises the 5'-start part (1). The 5'-start part (1) comprises from upstream to downstream and adjacent to each other the following elements: an N-outer polypeptide specification element (15), an N-outer splicing specification element (14), an N-inner polypeptide specification element (13), an N-inner splicing specification element (12) and an N-centered integration specification element (11). In Figure 5a, the N-outer polypeptide specification element (15, 130) can be completed to express the first marker (5). The N-outer splicing specification element (14) is a first 5'- cis-intein encoding sequence (122a). The N-inner polypeptide specification element (13, 131) can be completed to express the second marker (51). The N-inner splicing specification element (12) is a phase-specific 5'- intron sequence (121). The N-centered integration specification element (11) comprises a 5'-centered integrase specific integration site (110).

Every one of the integration strands shown in Figures 5ba and 5bb is an integration strand comprising a first (61) and a second DNA-sequence (62).

The first DNA-sequence (61) comprises from upstream to downstream and adjacent to each other the following elements: An N-outer polypeptide specification element (15), an N-outer splicing specification element (14), an N-inner polypeptide specification element (13) and an N-inner splicing specification element (12).

The second DNA-sequence (62) is a 3'-end part (2) and comprises, from upstream to downstream and adjacent to each other the following elements: A C-centered integration specification element (21), a C-inner splicing specification element (22), a C-inner polypeptide specification element (23), a C-outer splicing specification element (24) and a C-outer polypeptide specification element (25). The C-centered integration specification element (21) comprises a 3'-centered integrase specific integration site (210).

The N-outer polypeptide specification element (15) and the C-outer polypeptide specification element (25) on the same integration strand are compatible with each other and can express the first marker (5) when combined correctly. The N-outer splicing specification element (14) is a 5'- cis-intein encoding sequence (122b,c) and the C-outer splicing specification element (24) is a 3'- cis-intein encoding sequence (222b,c) which are compatible with each other and which can combine the N-outer polypeptide specification element (15) and the C-outer polypeptide specification element (25) on the same integration strand by intein splicing (42). Thereby, the first marker (5) can be expressed. The N-outer splicing specification element (14) is a second 5'- cis-intein encoding sequence (122b) and the C-outer splicing specification element (24) is a second 3'-cis-intein encoding sequence (222b) on the first integration strand as shown in Figure 5ba. The N-outer splicing specification element (14) is a third 5'-cis-intein encoding sequence (122c) and the C-outer splicing specification element (24) is a third 3'-cis-intein encoding sequence (222c) on the second integration strand as shown in Figure 5bb. Preferably, the first, second and third cis-intein encoding sequences (122a, 222a, 122b, 222b, 122c, 222c) are orthogonal to each other.

The N-inner polypeptide specification element (13) and the C-inner polypeptide specification element (23) on the same integration strand are incompatible with each other. The N-inner splicing specification element (12) is a phase-specific 5'- intron sequence (121) and the C-inner splicing specification element (22) is a phase-specific 3'- intron sequence (221) which are compatible with each other and can combine the incompatible N-inner polypeptide specification element (13) and the C-inner polypeptide specification element (23) on the same integration strand by intron splicing.

The N-inner polypeptide specification element (13, 132) of the integration stand according to Figure 5ba can be completed to express a third marker (52) and the C-inner polypeptide specification element (23, 231) of the same integration strand can be completed to express a second marker (51).

The N-inner polypeptide specification element (13, 133) of the integration stand according to Figure 5bb can be completed to express a fourth marker (53) and the C-inner polypeptide specification element (23, 232) of the same integration strand can be completed to express a third marker (52).

The second (51) and the third marker (52) are examples of inner markers. The N-inner splicing specification element (12) of a previous strand is compatible to the C-inner splicing specification element (22) of the following integration strand. The N-outer splicing specification element (14) of a previous strand is not compatible to C-outer splicing specification element (24) of the following integration strand. The previous strand can be the host strand or an integration strand.

Figure 5c depicts the host strand according to Figure 5a with integration strands according to Figures 5ba and 5bb integrated in the desired sequence. In this case the 5'-start part (1) of the host strand and the 3'-end part (2) of the fist integration strand depicted in Figure 5ba form a first expression cassette while the first DNA sequence (61) of the first integration strand depicted in Figure 5ba and the 3-end part (2) of the second integration strand depicted in Figure 5bb form a second expression cassette. In both expression cassettes, only intron splicing (41) is possible and the cis-intein encoding sequences are incompatible with each other. The intron splicing (41) combines the N-inner polypeptide specification element (13) of the previous strand with the C-inner polypeptide specification element (23) of the following strand. These elements are compatible with each other if the sequence of the strands is the desired one. Therefore the second and the third marker (51) and (52) can be expressed while the expression of the first marker (5) disappears in the case of successful integration.

Consequently, the fifth set can be used to integrate different genes of interest in a desired sequence. A missing or faulty integration can detected by the expression of the first marker. A successful integration can be detected by the expression of all inner markers occurring in the fifth set.

An example of a 5'-start part (1) and a 3'-end parts (2) as well as first DNA sequences (61) suitable to form a fifth set are given in the sequence listing SEQ ID No. 13, SEQ ID No. 14 and SEQ ID No. 15.

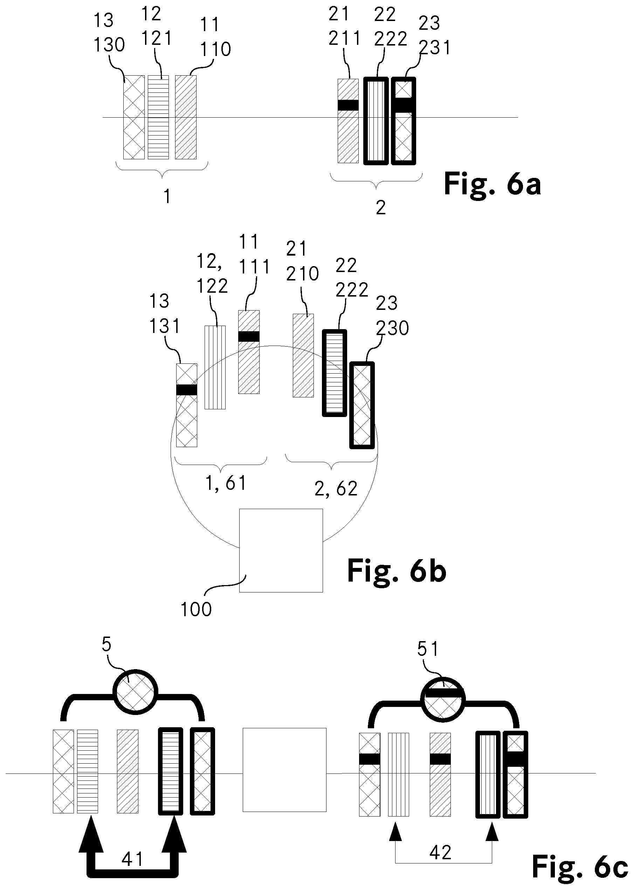

Figure 6a shows a host strand for a cassette exchange procedure. Figure 6b shows an integration strand as for the cassette exchange procedure. Figure 6c shows the host strand with the successfully integrated gene of interest (100) of the integration strand according to Figure 6b. The host strand according to Figure 6a and the integration strand according to Figure 6b form the sixth set. The sixth set can comprise more than one integration strand according to Figure 6b which differ only in the gene of interest 100. The sixth set allows to replace the integration strand integrated in the host strand by another integration strand.

The host strand of the sixth set comprises a 5'-start part (1) and a 3'-end part (2) which are not compatible with each other. The 5'-start parts (1) comprise, upstream to downstream and adjacent to each other, an N-inner polypeptide specification element (13), an N-inner splicing specification element (12) and an N-centered integration specification element (11). The 3'-end parts (2) comprise upstream to downstream and adjacent to each other, a C-centered integration specification element (21), a C-inner splicing specification element (22) and a C-inner polypeptide specification element (23). The N-inner polypeptide specification element (13, 130) can be completed to express a first marker (5). The N-inner splicing specification element (12) is a phase-specific 5'- intron sequence (121). The N-centered integration specification element (11) comprises a first 5'-centered integrase specific integration site (110). The N-centered integration specification element (110) which is incompatible with the C-centered integration specification element (21) comprising a second 3'-centered integrase specific integration site (211). The C-inner splicing specification element (22) is a 3'- cis-intein encoding sequence (222). The C-inner polypeptide specification element (23, 231) can be completed to express a second marker (51).

The integration strand of the sixth set depicted in Figure 6b comprises a 5'-start part (1) and a 3'-end part (2). The 5'-start parts (1) comprise, upstream to downstream and adjacent to each other, an N-inner polypeptide specification element (13), an N-inner splicing specification element (12) and an N-centered integration specification element (11). The 3'-end parts (2) comprise upstream to downstream and adjacent to each other, a C-centered integration specification element (21), a C-inner splicing specification element (22) and a C-inner polypeptide specification element (23). The gene of interest (100) is located between the 3'-end part (2) and the 5'-start part, whereby the 3'-end part (2) is located further upstream than the 5'-start part. The N-inner polypeptide specification element (13, 131) can be completed to express a second marker (51). The N-inner splicing specification element (12) is a 5'- cis-intein encoding sequence (122) which is compatible with the C-inner splicing specification element (22) of the host strand. The N-centered integration specification element (11) is a second 5'-centered integrase specific integration site (111) which is incompatible with the C-centered integration specification element (21) being a first 3'-centered integrase specific integration site (210). However, the second 5'-centered integrase specific integration site (111) of the integration strand is compatible with the second 3'-centered integrase specific integration site (211) of the host strand. The first 3'-centered integrase specific integration site (210) of the integration strand is compatible with the first 5'-centered integrase specific integration site (110) on the host strand. The C-inner splicing specification element (22) is a phase-specific 3'- intron sequence (221). The C-inner polypeptide specification element (23, 230) can be completed to express a second marker (51).

The first and the second centered integrase specific integration site (110, 210 and 111, 211) are in the embodiment shown in Figures 6a-c orthogonal to each other. The term "centered integrase specific integration site" denotes the 3'- centered integrase specific integration site and the compatible 5'-centered integrase specific integration site.

Consequently, the 3-end part (2) of the integration strand of the sixth set is compatible with the 5'-start part (1) of the host strand of the sixth set such that in the case of successful integration the first marker (5) is expressed if the intron splicing (41) occurred.

The 5'-start part (1) of the integration strand of the sixth set is compatible with the 3' -end part (2) of the host strand of the sixth set such that in the case of successful integration the second marker (51) is expressed if the intein splicing (42) occurred.

In another embodiment of the sixth set, the first and the second centered integrase specific integration site (110, 210 and 111, 211) are not orthogonal to each other, but the C-inner splicing specification element (22) of the 3'-end part is not compatible with the N-inner splicing specification element of the 5'-start part (12). Again, in the case of successful integration, the first and the second marker (5, 51) are expressed only if the intended splicing occurred. The intended splicing can only occur if compatible splicing specification elements (22) are combined because of the integration.

The sixth set can therefore be used in a cassette exchange process whereby a successful exchange can be detected by the expression of the first (5) and the second marker (51).

Furthermore, in this configuration the integration specification elements are well tolerated expression-wise.

An example of a 5'-start part (1) and a 3'-end part (2) to be arranged on the host respectively on the integration strands to form a sixth set are given in the sequence listings SEQ ID No. 16, SEQ ID No. 17, SEQ ID No. 18, SEQ ID No. 19.

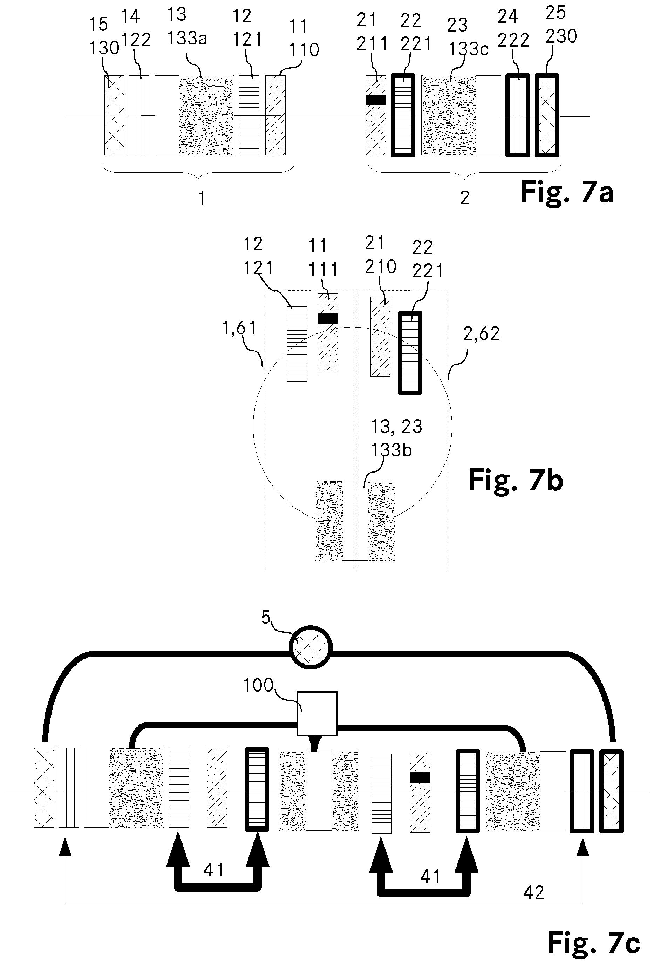

Figure 7a shows a host strand of a seventh set. Figure 7b shows an integration strand of the seventh set and Figure 7c shows the integration strand of Figure 7b after successful integration in the host strand according to Figure 7a. The seventh set is preferably used to study mutagenesis as it is in principle a cassette exchange procedure where only parts of the gene to be studied are exchanged.

The seventh set comprises one host strand and two or more integration strands. However, the integration strands differ only in the part of the gene to be studied (133b). Therefore, only one example is shown.

The host strand of the seventh set comprises a 5'-start part (1) and a 3-end part (2) which are not compatible with each other. The integration strands of the seventh set are suitable to replace each other at the desired location of the host strand.

The 5'-start part (1) of the host strand of the seventh set shown in Figure 7a comprises from upstream to downstream and adjacent to each other the following elements: an N-outer polypeptide specification element (15), an N-outer splicing specification element (14), an N-inner polypeptide specification element (13), an N-inner splicing specification element (12) and an N-centered integration specification element (11). In Figure 7a, the N-outer polypeptide specification element (15, 130) can be completed to express the first marker (5). The N-outer splicing specification element (14) is a 5'- cis-intein encoding sequence (122). The N-inner polypeptide specification element (13, 133a) can be completed to express the gene of interest (100).The N-inner splicing specification element (12) is a phase-specific 5'- intron sequence (121). The N-centered integration specification element (11) comprises a first 5'-centered integrase specific integration site (110).

The 3'-end part (2) of the host strand of the seventh set shown in Figure 7a comprises from upstream to downstream and adjacent to each other the following elements: a C-centered integration specification element (21), a C-inner splicing specification element (22), a C-inner polypeptide specification element (23), a C-outer splicing specification element (24) and an C-outer polypeptide specification element (25). In Figure 7a, the C-outer polypeptide specification element (25, 230) can be completed to express the first marker (5). The C-outer splicing specification element (24) is a 3'- cis-intein encoding sequence (222). The C-inner polypeptide specification element (23, 133c) can be completed to express the gene of interest (100). The C-inner splicing specification element (22) is a phase-specific 3'- intron sequence (221). The C-centered integration specification element (21) comprises a second 3'-centered integrase specific integration site (211).

Every one of the integration strands, such as the example shown in Figure 7b, of the seventh set comprises a 5'-start part (1) and a 3'-end part (2).

The 5'-start part (1) of the integration strand of the seventh set shown in Figure 7b comprises from upstream to downstream and adjacent to each other the following elements: an N-inner polypeptide specification element (13), an N-inner splicing specification element (12) and an N-centered integration specification element (11). In Figure 7b, the N-inner polypeptide specification element (13, 133b) can be comlpeted to express the gene of interest (100). The N-inner splicing specification element (12) is a phase-specific 5'- intron sequence (121). The N-centered integration specification element (11) is a second 5'-centered integrase specific integration site (111).

The 3'-end part (2) of the integration strand of the seventh set shown in Figure 7b comprises from upstream to downstream and adjacent to each other the following elements: a C-centered integration specification element (21), a C-inner splicing specification element (22), an C-inner polypeptide specification element (23). In Figure 7b, the C-inner polypeptide specification element (23, 133b) can be completed to express the gene of interest (100). The C-inner splicing specification element (22) is a phase-specific 3'- intron sequence (221). The C-centered integration specification element (21) is a first 3'-centered integrase specific integration site (210).

The N-inner polypeptide specification element (13, 133b) and the C-inner polypeptide specification element (13, 133b) are arranged adjacent to each other with an essentially arbitrate interface between the two parts. Therefore they are shown as one common sequence block in Figure 7b.

The 3'-end part (2) of the integration strand according to Figure 7b of the seventh set is compatible with the 5'-start part (1) of the host strand of the seventh set shown in Figure 7a. The 5'-start part (1) of the integration strand of the seventh set shown in Figure 7b is compatible with the 3'-end part (2) of the host strand of the seventh set shown in Figure 7a.

The N-inner polypeptide specification element (133a) of the host strand, the C-inner polypeptide specification element (133b) of the integration strand, the N-inner polypeptide specification element (133b) of the integration strand and the C-inner polypeptide specification element (133c) of the host strand are combined the gene of interest of the seventh set. In the case of successful integration of an integration strand of the seventh set into the host strand of the seventh set, a desired version of the gene of interest (100) is expressed as intron splicing (41) removes the N- and C-centered integration specification elements (11, 21). Intein splicing (42) allows to express the marker (5) by combining the N-outer polypeptide specification element (15, 130) and the C-outer polypeptide specification element (25, 230).

Therefore, the seventh set is used in a cassette exchange process whereby only a part of the gene of interest is exchanged and whereby a successful exchange can be detected by the expression of the outer marker and by the expression of the gene of interest.

An example of a 5'-start part (1) and a 3'-end part (2) to be arranged on the host respectively on the integration strands to form a sixth set are given in the sequence listings SEQ ID No. 20, SEQ ID No. 21, SEQ ID No. 22, SEQ ID No. 23, SEQ ID No. 24, SEQ ID No. 25.

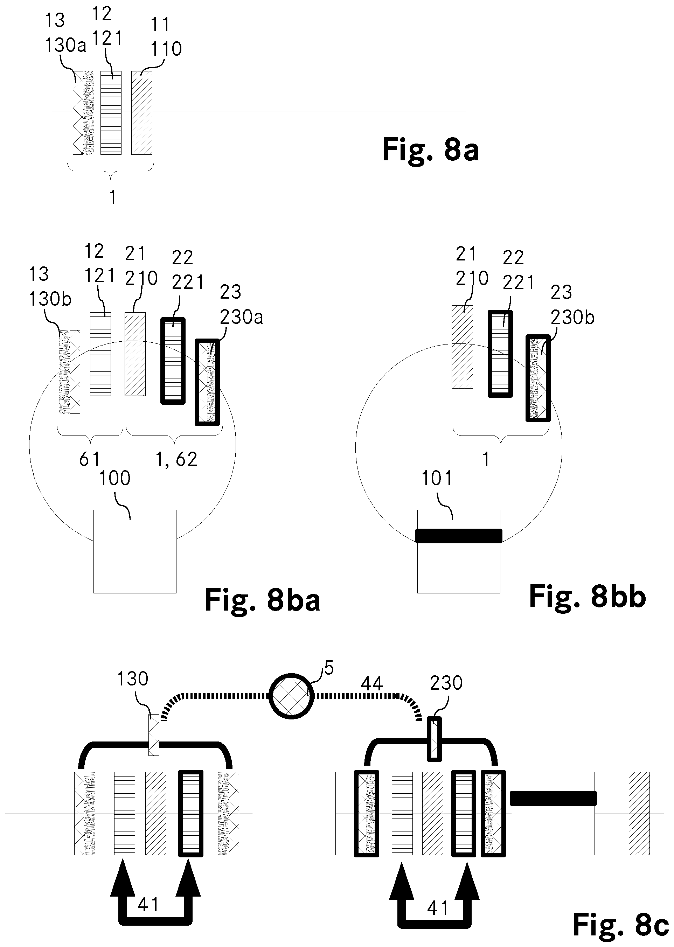

Figure 8a shows a host strand and Figure 8ba and 8bb two different integration strands of an eighth set. Figure 8c shows the result of the two integration strands integrated in the host strand in the desired sequence.

The host strand shown in Figure 8a of the eighth set comprises 5'-start part defining the desired location.

The 5'-start part (1) of the host strand of the eighth set shown in Figure 8a comprises from upstream to downstream and adjacent to each other the following elements: an N-inner polypeptide specification element (13), an N-inner splicing specification element (12) and an N-centered integration specification element (11). In Figure 8a, the N-inner polypeptide specification element (13, 130a) can be completed to express a first marker (5). The N-inner splicing specification element (12) is a phase-specific 5'- intron sequence (121). The N-centered integration specification element (11) comprises a 5'-centered integrase specific integration site (110).

The first integration strand shown in Figure 8ba of the eighth set comprises a 3'-end part (2) and a first DNA-sequence (61). The second integration strand shown in Figure 8bb of the eighth set comprises a 3'-end part (2).

The 3'-end part (2) of the first and the second integration strand of the eighth set shown in Figures 8ba and 8bb comprise from upstream to downstream and adjacent to each other the following elements: a C-centered integration specification element (21), a C-inner splicing specification element (22) and a C-inner polypeptide specification element (23). In Figures 8ba and 8bb, the C-inner polypeptide specification element (23, 230a and 230b) can be completed to express a first marker (5). The C-inner splicing specification element (22) is a phase-specific 3'- intron sequence (221). A gene of interest (100, 101) is arranged downstream, of the 3'-end part (2) in both integration strands. The C-centered integration specification element (21) comprises a 3'-centered integrase specific integration site (210).

The first DNA sequence (61) present in the first integration strand shown in Figure 8ba comprises a N-inner polypeptide specification element (13, 130b) and a N-inner splicing specification element (12, 121). The N-inner polypeptide specification element (13, 130b) can be completed to express a first marker (5). The N-inner splicing specification element (12) is a phase-specific 5'- intron sequence (121).

The N-inner polypeptide specification element (130a) of the host strand of the eighth set and the C-inner polypeptide specification element (230a) of the first integration strand of the eighth set can be combined to result in a first non-functional half (130) of a marker (5).

The N-inner polypeptide specification element (130b) of the first integration strand of the eighth set and the C-inner polypeptide specification element (230b) of the second integration strand of the eighth set can be combined to result in a second non-functional half (230) of a marker (5).

Upon successful integration of both integration strands of the eighth set into the host strand of the eighth set at the desired location, the first and the second non-functional half of a marker (5) can be expressed by intron splicing (41). The two non-functional halves can then be combined to express the marker (5). Either the marker (5) used in the eighth set comprises a trans-intein encoding sequence or the marker (5) is a protein or a polypeptide which is functional after the separately expressed two halves undergo a successful reconstitution. In the shown example, the first and the second non-functional half of the marker (5) comprise a 5'- respectively 3'- trans-intein encoding sequence. The detectable protein of the marker (5) is expressed once the two non-functional halves are combined by trans-intein protein splicing (44).

The eighth set is preferably used to integrate two or more genes of interest at one or more a desired locations on one or more independent integration strands. Figure 8a-c show the case of two integration strands being integrated in a desired sequence at a given location on a single integration strand. In all embodiments of the eight set, a successful integration can be detected by the expression of a single marker.

An example of a 5'-start part (1) and a 3'-end part (2) to be arranged on the host respectively on the integration strands to form an eighth set are given in the sequence listings SEQ ID No. 26, SEQ ID No. 27, SEQ ID No. 28, SEQ ID No. 29.

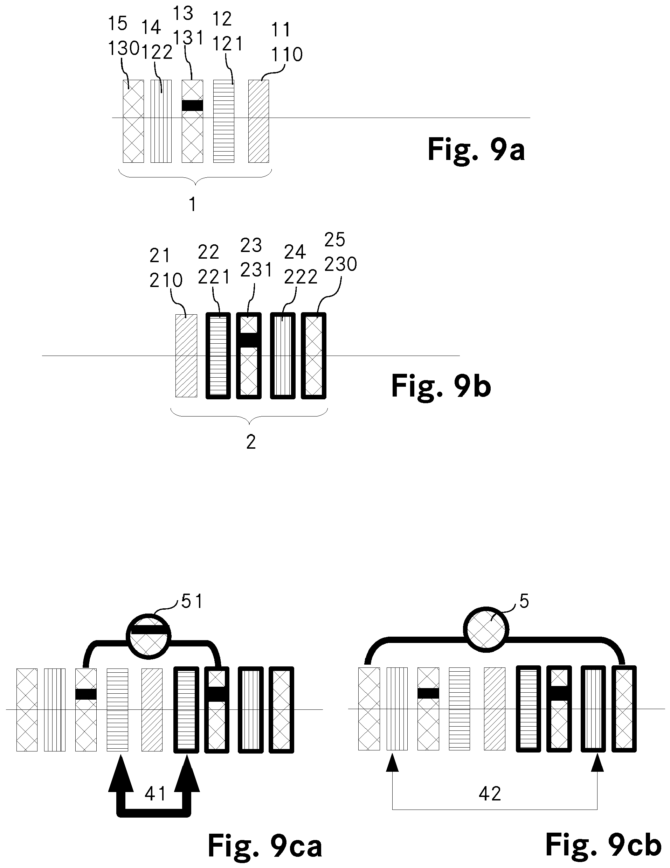

Figure 9a shows a host strand. Figure 9b shows an integration strand. Figures 9ca shows the result of an intron splicing applied to the host strand of Figure 9a after successful integration of the integration strand of Figure 9b. Figures 9cb shows the result of an intein splicing applied to the host strand of Figure 9a after successful integration of the integration strand of Figure 9b. As a marker is expressed either if an intron splicing occurs or if an intein splicing occurs of if both, intein and intron splicing occur, the host strand with the integrated integration strand as shown in Figures 9a, 9b, 9ca and 9cb form a logical OR-gate.

Figure 9a shows a host strand comprising a 5'-start part (1). The 5'-start part (1) comprises from upstream to downstream and adjacent to each other the following elements: an N-outer polypeptide specification element (15), an N-outer splicing specification element (14), an N-inner polypeptide specification element (13), an N-inner splicing specification element (12) and an N-centered integration specification element (11). In Figure 9a, the N-outer polypeptide specification element (15, 130) can be completed to express the first marker (5). The N-outer splicing specification element (14) is a 5'- cis-intein encoding sequence (122). The N-inner polypeptide specification element (13, 131) can be completed to express the second marker (51). The N-inner splicing specification element (12) is a phase-specific 5'- intron sequence (121). The N-centered integration specification element (11) comprises a 5'-centered integrase specific integration site (110).

Figure 9b shows an integration strand comprising a 3'-end part (2). The 3'-end part (2) comprises from upstream to downstream and adjacent to each other the following elements: a C-centered integration specification element (21), a C-inner splicing specification element (22), a C-inner polypeptide specification element (23), a C-outer splicing specification element (24) and a C-outer polypeptide specification element (25). In Figure 9b, the C-outer polypeptide specification element (25, 230) can be completed with the N-outer polypeptide specification element (15, 130) of the host strand to express the first marker (5). The C-outer splicing specification element (24) is a 3'- cis-intein encoding sequence (222). The C-outer splicing specification element (24) is compatible with the N-outer splicing specification element (14) of the host strand. The C-centered integration specification element (21) comprises a 3'-centered integrase specific integration site (210).

The C-inner polypeptide specification element (23, 231) can be completed with the N-inner polypeptide specification element (13, 131) of the host strand to express the second marker (51). The C-inner splicing specification element (22) is a phase-specific 3'- intron sequence (221) which is compatible with the N-inner splicing specification element (12) of the host strand.

After the successful integration an expression cassette results which comprises a nested arrangement of the inner splicing specification element, the second marker, the outer splicing specification element and the outer marker. The inner splicing specification elements are intron sequences, therefore intron splicing (41) combines the two halves of the second marker, such that an expressed second marker indicates a successful intron splicing. The outer splicing specification elements are intein encoding sequences. Therefore intein splicing (42) combines the two halves of the first marker, such that an expressed first marker indicates a successful intein splicing. If the conditions for intron or intein splicing are considered to be input values and the expression of any marker is considered to be an output, the expression cassette at hand is a logical OR gate. Furthermore, the splicing-dependent expression of the expression cassette can be controlled by two different splicing reactions.

An example of a 5'-start part (1) and a 3'-end part (2) to be arranged on the host respectively on the integration strands to form an OR-gate are given in the sequence listings SEQ ID No. 30, SEQ ID No. 31.

Further examples illustrate the use of the 5'-start parts and 3'-end parts of ultrashort expression cassettes according to the invention which do not need a C- respectively and N-inner splicing specification element:
SEQ ID No. 32 and SEQ ID No. 33 show an example where a GFP marker can be expressed on correct complementation of the expression cassette.
SEQ ID No. 34 and SEQ ID No. 35 show an example where a Cherry marker can be expressed on correct complementation of the expression cassette.
SEQ ID No. 36 and SEQ ID No. 37 show an example where a NeonGreen marker can be expressed on correct complementation of the expression cassette.
SEQ ID No. 38, SEQ ID No. 39 and SEQ ID No. 40 show an example where a GFP marker is split in a three non-functional parts. Upon correct integration, a first expression cassette consisting of the SEQ ID No. 38 and SEQ ID No. 39 can express a first non-functional half of the GFP Marker while a second expression cassette can be used to express a second non-functional half of the GFP Marker as shown in SEQ ID No. 40. As the GFP Marker as specified in these sequences is a split-marker, it will be expressed without the need for further splicing processes. This first and second non-functional halves of the GFP marker are preferably used in an eighth set according to the invention.

In all of the embodiments described above, the integration strands may carry more than one or only parts of genes of interest instead of a single gene of interest (100. 101, 102).

## Claims

1. A 5'-start part of an expression cassette comprising the 5'-start part and a 3'-end part,
a. wherein the 5'-start part of the expression cassette is arranged on a DNA-strand different from the one carrying the 3'-end part of the same expression cassette,
b. wherein the 3'-end part comprises a C-centered integration specification element (CC-ISE), a C-inner polypeptide specification element (CI-PSE), and preferably a C-inner splicing specification element (CI-SSE),
c. wherein the 5'-start part comprises a N-centered integration specification element (NC-ISE), a N-inner polypeptide specification element (NI-PSE), and preferably a N-inner splicing specification element (NI-SSE),
d. wherein the N-centered integration specification element (NC-ISE)
i. is compatible with the C-centered integration specification element (CC-ISE) of the expression cassette and
ii. comprises a 5'-centered integrase specific integration site and
e. wherein the N-inner polypeptide specification element (NI-PSE)
i. is compatible with the C-inner polypeptide specification element (CI-PSE) of the expression cassette and
ii. the polypeptide encoded by the N-inner polypeptide specification element (NI-PSE) is itself non-functional and
iii. the polypeptide encoded by the N-inner polypeptide specification element (NI-PSE) can be expressed from the expression cassette upon complementation by the C-inner polypeptide specification element (CI-PSE) and
iv. the N-inner polypeptide specification element (NI-PSE) is operably linked to the N-inner splicing specification element (NI-SSE), in the preferred embodiment of a 5'-start part comprising the N-inner splicing specification element (NI-SSE),
v. the N-inner polypeptide specification element (NI-PSE) is operably linked to the N-centered integration specification element (NC-ISE), in embodiments of the 5'-start part not comprising the N-inner splicing specification element (NI-SSE),
f. wherein preferably the N-inner splicing specification element (NI-SSE)
i. is compatible with the preferably existing C-inner splicing specification element (CI-SSE) of the expression cassette and
ii. is by itself splicing-incompetent and
iii. can be rendered functional upon complementation by the C-inner splicing specification element (CI-SSE) and
iv. comprises a phase-specific 5'- intron sequence or a 5'- cis-intein encoding sequence
v. whereby the N-inner splicing specification element (NI-SSE) is operably linked to the N-centered integration specification element (NC-ISE).

2. A 3'-end part of an expression cassette comprising a 5'-start part and the 3'-end part,
a. wherein the 5'-start part of the expression cassette is arranged on a DNA-strand different from the one carrying the 3'-end part of the same expression cassette,
b. wherein the 3'-end part comprises a C-centered integration specification element (CC-ISE), a C-inner polypeptide specification element (CI-PSE), and preferably a C-inner splicing specification element (CI-SSE),
c. wherein the 5'-start part comprises a N-centered integration specification element (NC-ISE), a N-inner polypeptide specification element (NI-PSE), and preferably a N-inner splicing specification element (NI-SSE),
d. wherein the C-centered integration specification element (CC-ISE)
i. is compatible with the N-centered integration specification element (NC-ISE) of the expression cassette and
ii. comprises a 3'-centered integrase specific integration site and
e. wherein the C-inner polypeptide specification element (CI-PSE)
i. is compatible with the N-inner polypeptide specification element (NI-PSE) of the expression cassette and
ii. the polypeptide encoded by the C-inner polypeptide specification element (CI-PSE) is itself non-functional and
iii. the polypeptide encoded by the C-inner polypeptide specification element (CI-PSE) can be expressed from the expression cassette upon complementation by the N-inner polypeptide specification element (NI-PSE) and
iv. whereby the C-inner polypeptide specification element (CI-PSE) is operably linked to the C-inner splicing specification element (CI-SSE), in the preferred embodiment of a 3'-end part comprising the C-inner splicing specification element (CI-SSE),
v. whereby the C-inner polypeptide specification element (CI-PSE) is operably linked to the C-centered integration specification element (CC-ISE), in embodiments of the 3'-end part not comprising the C-inner splicing specification element (CI-SSE),
f. wherein preferably the C-inner splicing specification element (CI-SSE)
i. is compatible with the preferably existing N-inner splicing specification element (NI-SSE) of the expression cassette and
ii. is by itself splicing-incompetent and
iii. can be rendered functional upon complementation by the N-inner splicing specification element (NI-SSE) and
iv. comprises a phase-specific 3'- intron sequence or a 3'- cis-intein encoding sequence,
v. whereby the C-inner splicing specification element (CI-SSE) is operably linked to the C-centered integration specification element (CC-ISE).

3. The 3'-end part of an expression cassette according to claim 2, wherein the C-inner splicing specification element (CI-SSE) comprises two splicing specifications which are the phase-specific 3'- intron sequence, and the 3'- cis-intein encoding sequence.

4. A host strand, which is a DNA-strand comprising
a. one, preferably two or more, 5'-start parts according to claim 1 each one defining a location to integrate one or more genes of interest and
b. preferably,
i. downstream of the 5'-start part,
ii. a 3'-end part according to any one of claims 2-3 which is incompatible with the 5'-start part.

5. An integration strand, which is a DNA-strand comprising a 3'-end part according to any one of claims 2-3 to be integrated in a host strand comprising a compatible 5'-start part, whereby the integration strand comprises
a. either one or more genes of interest outside of the 3'- end part or
b. the one or more genes of interest form at least partially the C-inner polypeptide specification element.

6. A first set of a host strand according to claim 4 and an integration strand according to claim 5 comprising a gene of interest to be integrated in the host strand, wherein
a. the 5'-start part on the host strand.
i. comprises the N-inner splicing specification element (NI-SSE)
ii. whereby the N-inner splicing specification element (NI-SSE) of the host strand is made of the 5'- cis-intein encoding sequence arranged upstream and adjacent to the phase-specific 5'- intron sequence and wherein
iii. the N-inner polypeptide specification element can express a marker when completed with a compatible C-inner polypeptide specification element.
b. and the 3'-end part on the integration strand
i. comprises the C-inner splicing specification element (CI-SSE)
ii. whereby the C-inner splicing specification element (CI-SSE) of the integration strand is made of the phase-specific 3'- intron sequence compatible with the phase-specific 5'- intron sequence on the host strand, which is arranged adjacent to and upstream of the 3'- cis-intein encoding sequence compatible with the 5'-cis-intein encoding sequence on the host strand.
c. whereby the 3'-centered integrase specific integration site of the integration strand is compatible with the 5'-centered integrase specific integration site on the host strand and
d. whereby the C-inner polypeptide specification element of the integration strand can express the marker when completed with the compatible N-inner polypeptide specification element of the host strand.

7. A second set of a host strand according to claim 4 and two or more integration strands according to claim 5, which should be integrated in the host strand in a desired sequence,
a. wherein the 5'-start part of the host strand
i. comprises the N-inner splicing specification element (NI-SSE).
ii. the N-inner polypeptide specification element is compatible with the C-inner polypeptide specification element of the following integration strand
iii. The N-inner splicing specification element (NI-SSE) is compatible with the C-inner splicing specification element (CI-SSE) of the following integration strand.
b. The integration strands comprises a second DNA-sequence and preferably a first DNA-sequence, whereby
i. the first DNA-sequence comprises
(A) a N-inner splicing specification element (NI-SSE), which is compatible with the C-inner splicing specification element of the following strand
(B) and a N-inner polypeptide specification element (NI-PSE) which is compatible with the C-inner polypeptide specification element of the following strand, and
(C) whereby the N-inner polypeptide specification element is arranged adjacent and upstream of the N-inner splicing specification element (NI-SSE).
ii. the second DNA-sequence is a 3'-end-part according to any one of claims 1-2, comprising a C-inner splicing specification element, wherein
(A) the 3'-centered integrase specific integration site is compatible with the 5'-centered integrase specific integration site and
(B) the C-inner splicing specification element (CI-SSE) is compatible with the N-inner splicing specification element (NI-SSE) of the previous strand.
(C) the C-inner polypeptide specification element is compatible with the N-inner polypeptide specification element of the previous strand.
c. wherein all C-centered integration specification elements are compatible with all N-centered integration specification element occurring in the second set and
d. the combination of C-inner splicing specification elements (CI-SSE) and C-inner polypeptide specification element of a given strand is only compatible with the combination of the N-inner polypeptide specification element and N-inner splicing specification elements (NI-SSE) of following strand and
e. wherein the previous strand is the host strand or the integration strand which is located adjacent to and upstream of the strand at hand while the following strand is the integration strand located adjacent to and downstream of the strand at hand in the desired sequence.

8. A third set comprises two or more host strands according to claim 4 and two or more integration strands according to claim 5
a. whereby every one of the host strands of the third set comprises a 5'-start part which comprises an N-inner splicing specification element (NI-SSE) and
b. every one of the integration strands of the third set comprises a 3'-end part which comprises an C-inner splicing specification element (CI-SSE) and genes of interest to be integrated in the host strand
c. whereby the combination of C-inner splicing specification element (CI-SSE) and C-inner polypeptide specification element of an integration strand is only compatible with the combination of N-inner polypeptide specification element and N-inner splicing specification element (NI-SSE) of the host strand in which it should be integrated.

9. A fourth set comprises a host strand according to claim 4 and two or more integration strands according to claim 5 to be integrated at a desired integration site in a desired sequence, whereby
a. the 5'-start part of the host strand comprises a N-inner splicing specification element (NI-SSE) and whereby
b. Every one of the integration strands comprises a first and a second DNA-sequence whereby
i. the first DNA-sequence comprises
(A) a N-inner splicing specification element (NI-SSE),
(B) and a N-inner polypeptide specification element (NI-PSE) and
(C) whereby the N-inner polypeptide specification element is arranged adjacent and upstream of the N-inner splicing specification element (NI-SSE).
(D) the second DNA-sequence is a 3'-end part comprising a C-inner splicing specification element, whereby
• either the C-inner splicing specification element of the second DNA-sequence comprises a first and a second 3'-inner splicing specification and the N-inner splicing specification element (NI-SSE) of the first DNA-sequence of a previous integration strand or of the host strand is compatible to the first 3'-inner splicing specification of the second DNA sequence of the following integration strand
• or the N-inner splicing specification element of the first DNA-sequence and of the host strand comprises a first and a second 5'-inner splicing specification and the C-inner splicing specification element (CI-SSE) of the second DNA-sequence of a following integration strand is compatible to the second 5'-inner splicing specification of the first DNA sequence of the previous integration strand or the host strand
c. whereby all C-inner polypeptide specification elements of any integration strand are compatible with every one of the N-inner polypeptide specification element of the host strand or any integration strand of the fourth set
d. wherein the previous strand is the host strand or the integration strand which is located adjacent to and upstream of the strand at hand while the following strand is the integration strand located adjacent to and downstream of the strand at hand in the desired sequence.

10. A fifth set comprises a host strand according to claim 4 and two or more integration strands according to claim 5 to be integrated at a desired integration site in a desired sequence whereby
a. the 5'-start part of the host strand comprises
i. the N-inner splicing specification element (NI-SSE),
ii. a N-outer splicing specification element and
iii. a N-outer polypeptide specification element
iv. whereby the N-outer splicing specification element (NO-SSE)
(A) comprises a 5'- cis-intein encoding sequence
(B) is operably linked to the N-inner polypeptide specification element (NI-PSE)
(C) is by itself splicing-incompetent and
(D) can be rendered functional upon complementation by a compatible C-outer splicing specification element (CO-SSE)
v. whereby the N-outer polypeptide specification element (NO-PSE)
(A) is operably linked to the N-outer splicing specification element (NO-SSE)
(B) and whereby the polypeptide encoded by the N-outer polypeptide specification element (NO-PSE)
• is by itself non-functional
• and can be expressed from the expression cassette upon complementation by a compatible C-outer polypeptide specification element (CO-PSE)
b. whereby every one of the integration strands comprises a first and a second DNA-sequence
i. wherein the first DNA-sequence comprises, from upstream to downstream and adjacent to each other,
(A) an N-outer polypeptide specification element and
(B) an N-outer splicing specification element and
(C) an N-inner polypeptide specification element (NI-PSE) and
(D) an N-inner splicing specification element (NI-SSE)
ii. and wherein the second DNA-sequence is a 3'-end-part according to any one of claims 1-2, comprising the C-inner splicing specification element, and
(A) a C-outer splicing specification element and
(B) a C-outer polypeptide specification element
(C) whereby the C-outer splicing specification element (CO-SSE)
• comprises a 3'- cis-intein encoding sequence
• is operably linked to the C-inner polypeptide specification element (Cl-PSE)
• is by itself splicing-incompetent and
• can be rendered functional upon complementation by a compatible N-outer splicing specification element (NO-SSE)
(D) whereby the C-outer polypeptide specification element (CO-PSE)
• is operably linked to the C-outer splicing specification element (CO-SSE)
• and whereby the polypeptide encoded by the C-outer polypeptide specification element (CO-PSE)
- is by itself non-functional
- and can be expressed from the expression cassette upon complementation by a compatible N-outer polypeptide specification element (NO-PSE)
c. and wherein the N-inner splicing specification element (NI-SSE) of a previous strand is compatible to C-inner splicing specification element (CI-SSE) of the following integration strand
d. wherein the N-inner polypeptide specification element (NI-PSE) of a previous strand is only compatible with the C-inner polypeptide specification element (CI-PSE) of the following strand, resulting in the case of correct integration, in the expression of an inner marker
e. and wherein the N-outer splicing specification element (NO-SSE) of a previous strand is not compatible to C-outer splicing specification element (CO-SSE) of the following integration strand while the N- and C-outer splicing specification element (CO-SSE) of the same strand are compatible, and
f. wherein the N-outer polypeptide specification element (NO-PSE) and the C-outer polypeptide specification element (CO-PSE) on the same strand are compatible resulting in the expression of an outer marker in the absence of an integration in the host strand
g. wherein the previous strand is the host strand or the integration strand which is located adjacent to and upstream of the strand at hand while the following strand is the integration strand located adjacent to and downstream of the strand at hand in the desired sequence.

11. A sixth set comprises a host strand according to claim 4 and two or more integration strands according to claim 5, which are suitable to replace each other at the desired location of the host strand, whereby
a. the host strand comprises a 5'-start part and a 3'-end part according to anyone of claims 2-3 which are not compatible with each other
b. every one of the integration strands comprises a 5'-start part according to claim 1 and a 3'-end part and genes of interest arranged between the 5'-start part and the 3'-end part and
c. wherein the different integration strands of the sixth set differ only in the genes of interest while all 5'-start parts and all a 3-end parts are the same
d. wherein the 3'-end parts of the integration strands of the sixth set are compatible with the 5'-start part of the host strand of the sixth set such that in the case of successful integration a first marker can be expressed and
e. wherein the 5'-start part of the integration strands of the sixth set are compatible with the 3-end part of the host strand of the sixth set such that in the case of successful integration a second marker can be expressed

12. A seventh set comprises a host strand according to claim 4 and two or more integration strands according to claim 5 which are suitable to replace each other at the desired location of the host strand, whereby
a. the host strand comprises a 5'-start part and a 3'-end part according to anyone of claims 2-3 which are not compatible with each other
b. and every one of the integration strands comprises a 5'-start part and a 3'-end part, which are not compatible with each other, and genes of interest arranged between the 5'-start part and the 3'-end part and
c. wherein the different integration strands differ only in the genes of interest while all 5'-start parts and all a 3'-end parts are the same,
d. wherein the 3'-end parts of the integration strands are compatible with the 5'-start part of the host strand and
e. wherein the 5'-start part of the integration strands are compatible with the 3'-end part of the host strand
f. whereby the N-inner polypeptide specification element (NI-PSE) of the host strand, the C-inner polypeptide specification element (CI-PSE) of the integration strand, the N-inner polypeptide specification element (NI-PSE) of the integration strand and the C-inner polypeptide specification element (CI-PSE) of the host strand are combined the gene of interest of the seventh set such that in the case of successful integration of an integration strand of the seventh set into the host strand of the seventh set, a desired version of the gene of interest is expressed
g. whereby, preferably, the host strand comprises
i. a N-outer polypeptide specification element (NO-PSE) arranged upstream of a N-outer splicing specification element (NO-SSE) which is arranged upstream and operably linked to the N-inner polypeptide specification element (NI-PSE) of the host strand
ii. and a C-outer splicing specification element (CO-SSE) which is arranged downstream and operably linked to the C-inner polypeptide specification element (CI-PSE) of the host strand which is arranged upstream of a C-outer polypeptide specification element (CO-PSE)
iii. whereby the N- and C-outer splicing specification element (NO-SSE, CO-SSE) of the host strand are compatible with each other and
iv. whereby the N- and C-outer polypeptide specification element (NO-PSE, CO-PSE) of the host strand are compatible with each other
v. such that in the case of successful integration of an integration strand of the seventh set into the host strand of the seventh set the outer marker is expressed.

13. An eighth set comprises
i. a 5'-start part,
ii. a first DNA sequence comprising a N-inner splicing specification element (NI-SSE) and a N-inner polypeptide specification element (NI-PSE) and
iii. two 3'-end parts
b. wherein the 5'-start part and one of the two 3'end parts are part of a first expression cassette
c. and wherein the first DNA sequence and the other one of the two 3'end parts are part of a second expression cassette
d. and wherein the N-inner polypeptide specification element (NI-PSE) of the 5'-start part of the first expression cassette and the C-inner polypeptide specification element (Cl-PSE) of the 3'-end part of the first expression cassette can be combined to result in a first non-functional half of a marker
e. and wherein the N-inner polypeptide specification element (NI-PSE) of the first DNA sequence of the second expression cassette and the C-inner polypeptide specification element (CI-PSE) of the 3'-end part of the second expression cassette can be combined to result in a second non-functional half of a marker
f. and whereby either
i. the first non-functional half of the marker comprises a 5'-trans-intein encoding sequence and the second non-functional half of the marker comprises a 3'-trans-intein encoding sequence
or
ii. the marker (5) is a split marker which can be expressed functionally even if its encoding sequences are translated separately,
g. whereby upon successful expression of the first and the second expression cassette of the eighth set, the first and the second non-functional half of a marker can be expressed to result in a functional marker.

14. A ninth set comprises a host strand according to claim 4 and an integration strand according to claim 5 to be integrated,
a. whereby the 5'-start part of the host strand comprises
i. the N-inner splicing specification element (NI-SSE),
ii. a N-outer splicing specification element and
iii. a N-outer polypeptide specification element
iv. whereby the N-outer splicing specification element (NO-SSE)
(A) comprises a 5'- cis-intein encoding sequence
(B) is operably linked to the N-inner polypeptide specification element (NI-PSE)
(C) is by itself splicing-incompetent and
(D) can be rendered functional upon complementation by a compatible C-outer splicing specification element (CO-SSE)
v. whereby the N-outer polypeptide specification element (NO-PSE)
(A) is operably linked to the N-outer splicing specification element (NO-SSE)
(B) and whereby the polypeptide encoded by the N-outer polypeptide specification element (NO-PSE)
• is by itself non-functional
• and can be expressed from the expression cassette upon complementation by a compatible C-outer polypeptide specification element (CO-PSE)
b. and whereby the a 3'-end part of the integration strand comprises
i. the C-inner splicing specification element, and
ii. a C-outer splicing specification element and
iii. a C-outer polypeptide specification element
iv. whereby the C-outer splicing specification element (CO-SSE)
(A) comprises a 3'- cis-intein encoding sequence
(B) is operably linked to the C-inner polypeptide specification element (CI-PSE)
(C) is by itself splicing-incompetent and
(D) can be rendered functional upon complementation by a compatible N-outer splicing specification element (NO-SSE)
v. whereby the C-outer polypeptide specification element (CO-PSE)
(A) is operably linked to the C-outer splicing specification element (CO-SSE)
(B) and whereby the polypeptide encoded by the C-outer polypeptide specification element (CO-PSE)
• is by itself non-functional
• and can be expressed from the expression cassette upon complementation by a compatible N-outer polypeptide specification element (NO-PSE)
c. whereby the N-centered integration specification element (NC-ISE) of the host strand is compatible to the C-centered integration specification element (CC-ISE) of the integration strand and
d. whereby the N-inner splicing specification element (NI-SSE) of the host strand is compatible to the C-inner splicing specification element (CI-SSE) of the integration strand
e. and whereby the N-inner polypeptide specification element (NI-PSE) of the host strand is compatible with the C-inner polypeptide specification element (CI-PSE) of the integration strand, resulting in the case of correct integration, in the expression of an inner marker
f. and whereby the N-outer splicing specification element (NO-SSE) of the host strand is compatible to the C-outer splicing specification element (CO-SSE) of the integration strand
g. and whereby the N-outer polypeptide specification element (NO-PSE) of the host strand is compatible with the C-outer polypeptide specification element (CO-PSE) of the integration strand, resulting in the case of correct integration, in the expression of an outer marker.

15. Use of the 5'-start part according to claim 1 and the 3'-end part according to claim 2 of an expression cassette to produce a genetically modified cell, wherein the 5'-start part and the 3'-end part are preferably part of a host strand according to claim 4 and an integration strand according to claim 5 which are particularly preferably part of a first set according to claim 6, a second set according to claim 7, a third set according to claim 8, a fourth set according to claim 9, a fifth set according to claim 10, a sixth set according to claim 11, a seventh set according to claim 12, an eighth set according to claim 13 or a ninth set according to claim 14.


SEQUENCE LISTING

<110> Anaxon AG

<120> Deterministic orthogonally specified integration sites

<130> SEQ ID NO: 1

<160> 40

<170> BiSSAP 1.3.6

<210> 1
<211> 647
<212> DNA
<213> Homo sapiens

<220>
<223> Fig. 1 Sequence

<400> 1
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc ctttcatacg agaccgagat cctgactgtc 300

gagtacggat tgcttcctat cggcaaaatc gtggagaaga ggattgaatg taccgtctat 360

tcagtcgata ataatgggaa catctacaca cagcccgtgg ctcaatggca cgacagagga 420

gagcaggaag tttttgaata ctgtctcgag gacggatccc tcatccgcgc tactaaagat 480

cataagttta tgaccgtgga cggccagatg ctgccaattg acgaaatttt tgaacgagag 540

ctggtgagtt gacctcagta acccaagtga gatcccaggg tgctgggaca ggaggtctgg 600

aagttcctat tccgaagttc ctattctcta gaaagtatag gaacttc 647

<210> 2
<211> 1038
<212> DNA
<213> Homo sapiens

<400> 2
gaagttccta ttccgaagtt cctattctct agaaagtata ggaacttctc ctgggggct 60

ccgctggtca ctcacccact ctccctcccc gctcctcgtc cctcctcctc ctcccccctg 120

ctcctccccc atcatgttct ccaggatctg atgagagtcg acaaccttcc aaacattaag 180

atcgctacgc ggaagtacct ggggaaacag aacgtctacg acataggtgt ggagcgcgat 240

cacaactttg ctctgaaaaa tggatttatc gccagcaact gcatctcccg ccgtgcacag 300

ggtgtcacgt tgcaagacct gcctgaaacc gaactgcccg ctgttctgca gccggtcgcg 360

gaggccatgg atgcgatcgc tgcggccgat cttagccaga cgagcgggtt cggcccattc 420

ggaccgcaag gaatcggtca atacactaca tggcgtgatt tcatatgcgc gattgctgat 480

ccccatgtgt atcactggca aactgtgatg gacgacaccg tcagtgcgtc cgtcgcgcag 540

gctctcgatg agctgatgct ttgggccgag gactgccccg aagtccggca cctcgtgcac 600

gcggatttcg gctccaacaa tgtcctgacg gacaatggcc gcataacagc ggtcattgac 660

tggagcgagg cgatgttcgg ggattcccaa tacgaggtcg ccaacatctt cttctggagg 720

ccgtggttgg cttgtatgga gcagcagacg cgctacttcg agcggaggca tccggagctt 780

gcaggatcgc cgcggctccg ggcgtatatg ctccgcattg gtcttgacca actctatcag 840

agcttggttg acggcaattt cgatgatgca gcttgggcgc agggtcgatg cgacgcaatc 900

gtccgatccg gagccgggac tgtcgggcgt acacaaatcg cccgcagaag cgcggccgtc 960

tggaccgatg gctgtgtaga agtactcgcc gatagtggaa accgacgccc cagcactcgt 1020

ccgagggcaa aggaatag 1038

<210> 3
<211> 422
<212> DNA
<213> Homo sapiens

<400> 3
atgagcgagc tgattaagga gaacatgcac atgaagctgt acatggaggg caccgtggac 60

aaccatcact tcaagtgcac atccgagggc gaaggcaagc cctacgaggg cacccagacc 120

atgagaatca aggtggtcga gggcggccct ctccccttcg ccttcgacat cctggctact 180

agcttcctct acggcagcaa gaccttcatc aaccacaccc agggcatccc cgacttcttc 240

aagcagtcct tccctgaggg cttcacatgg gagagagtca ccacatacga agacgggggc 300

gtgctgaccg ctacccaggt gagttgacct cagtaaccca agtgagatcc cagggtgctg 360

ggacaggagg tctggaagtt cctattccga agttcctatt ctctagaaag tataggaact 420

tc 422

<210> 4
<211> 528
<212> DNA
<213> Homo sapiens

<400> 4
   gaagttccta ttccgaagtt cctattctct agaaagtata ggaacttctc ctggggggct 60
   ccgctggtca ctcacccact ctccctcccc gctcctcgtc cctcctcctc ctcccccctg 120
   ctcctccccc atcatgttct ccaggacacc agcctccagg acggctgcct catctacaac 180
   gtcaagatca gaggggtgaa cttcacatcc aacggccctg tgatgcagaa gaaaacactc 240
   ggctgggagg ccttcaccga gacgctgtac cccgctgacg gcggcctgga aggcagaaac 300
   gacatggccc tgaagctcgt gggcgggagc catctgatcg caaacatcaa gaccacatat 360
   agatccaaga aacccgctaa gaacctcaag atgcctggcg tctactatgt ggactacaga 420
   ctggaaagaa tcaaggaggc caacaacgag acctacgtcg agcagcacga ggtggcagtg 480
   gccagatact gcgacctccc tagcaaactg gggcacaagc ttaattaa 528

<210> 5
<211> 621
<212> DNA
<213> Homo sapiens

<400> 5
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc atctcccgcc gtgcacaggg tgtcacgttg 300

gagtacggat tgcttcctat cggcaaatcc gtggagaaga ggattgaatg taccgtctat 360

tcagtcgata ataatgggaa catctacaca cagcccgtgg ctcaatggca cgacagagga 420

gagcaggaag tttttgaata ctgtctcgag gacggatccc tcatccgcgc tactaaagat 480

cataagttta tgaccgtgga cggccagatg ctgccaattg acgaaatttt tgaacgagag 540

ctggatctga tgagagtcga caaccttcca aacgaagttc ctattccgaa gttcctattc 600

tctagaaagt ataggaactt c 621

<210> 6
<211> 434
<212> DNA
<213> Homo sapiens

<400> 6
atggtgagca agggcgaggc agtgatcaag gagttcatgc ggttcaaggt gcacatggag 60

ggctccatga acggccacga gttcgagatc gagggcgagg gcgagggccg cccctacgag 120

ggcacccaga ccgccaagct gaaggtgacc aagggtggcc ccctgccctt ctcctgggac 180

atcctgtccc ctcagttcat gtacggctcc agggccttca ccaagcaccc cgccgacatc 240

cccgactact ataagcagtc cttccccgag ggcttcaagt gggagcgcgt gatgaacttc 300

gaggacggcg gcgccgtgac cgtgacccag gtgagttgac ctcagtaacc caagtgagat 360

cccagggtgc tgggacagga ggtctggaag ttcctattcc gaagttccta ttctctagaa 420

agtataggaa cttc                                                    434

<210> 7
<211> 513
<212> DNA
<213> Homo sapiens

<400> 7
gaagttccta ttccgaagtt cctattctct agaaagtata ggaacttctc ctgggggct      60

ccgctggtca ctcacccact ctccctcccc gctcctcgtc cctcctcctc ctcccccctg     120

ctcctccccc atcatgttct ccaggacacc tccctggagg acggcaccct gatctacaag    180

gtgaagctcc gaggcaccaa cttccctcct gacggccccg taatgcagaa gaagacaatg    240

ggctgggaag cgtccaccga gcggttgtac cccgaggacg gcgtgctgaa gggcgacatt    300

aagatggccc tgcgcctgaa ggacggcggc cgctacctgg cggacttcaa gaccacctac    360

aaggccaaga agcccgtgca gatgcccggc gcctacaacg tagaccgcaa gttggacatc    420

acctcccaca acgaggacta caccgtggtg gaacagtacg aacgctccga gggccgccac    480

tccaccggcg gcatggacga gctgtacaag taa                                 513

<210> 8
<211> 482
<212> DNA
<213> Homo sapiens

<400> 8
atggtgagca agggcgagga ggataacatg gcctctctcc cagcgacaca tgagttacac     60

atctttggct ccatcaacgg tgtggacttt gacatggtgg gtcagggcac cggcaatcca    120

aatgatggtt atgaggagtt aaacctgaag tccaccaagg gtgacctcca gttctccccc    180

tggattctgg tccctcatat cgggtatggc ttccatcagt acctgcccta ccctgacggg    240

atgtcgcctt tccaggccgc catggtagat ggctccggct accaagtcca tcgcacaatg    300

cagtttgaag atggtgcctc ccttactgtt aactaccgct acacctacga gggaagccac    360

atcaaaggag aggcccaggt gagttgacct cagtaaccca agtgagatcc cagggtgctg    420

ggacaggagg tctggaagtt cctattccga agttcctatt ctctagaaag tataggaact    480

tc                                                                   482

<210> 9
<211> 477
<212> DNA
<213> Homo sapiens

<400> 9

gaagttccta ttccgaagtt cctattctct agaaagtata ggaacttctc ctggggggct 60

ccgctggtca ctcacccact ctccctcccc gctcctcgtc cctcctcctc ctcccccctg 120

ctcctccccc atcatgttct ccaggtgaag gggactggtt tccctgctga cggtcctgtg 180

atgaccaact cgctgaccgc tgcggactgg tgcaggtcga agaagactta ccccaacgac 240

aaaaccatca tcagtacctt taagtggagt tacaccactg gaaatggcaa gcgttaccgg 300

agcactgcgc ggaccaccta cacctttgcc aagccaatgg cggctaacta tctgaagaac 360

cagccgatgt acgtgttccg taagacggag ctcaagcact ccaagaccga gctcaacttc 420

aaggagtggc aaaaggcctt taccgatgtg atgggcatgg acgagctgta caagtaa 477

<210> 10
<211> 632
<212> DNA
<213> Homo sapiens

<400> 10
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc atctcccgcc gtgcacaggg tgtcacgttg 300

caagacctgc ctgaaaccga actgcccgct gttctgcagc cggtcgcgga ggccatggat 360

gcgatcgctg cggccgatct tagccagacg agcgggttcg gcccattcgg accgcaagga 420

atcggtcaat acactacatg gcgtgatttc atatgcgcga ttgctgatcc ccatgtgtat 480

cactggcaaa ctgtgatgga cgacaccgtc agtgcgtccg tcgcgcaggt gagttgacct 540

cagtaaccca agtgagatcc cagggtgctg ggacaggagg tctggaagtt cctattccga 600

agttcctatt ctctagaaag tataggaact tc 632

<210> 11
<211> 1038
<212> DNA
<213> Homo sapiens

<400> 11
gaagttccta ttccgaagtt cctattctct agaaagtata ggaacttctc ctggggggct 60

ccgctggtca ctcacccact ctccctcccc gctcctcgtc cctcctcctc ctcccccctg 120

ctcctccccc atcatgttct ccaggatctg atgagagtcg acaaccttcc aaacattaag 180

atcgctacgc ggaagtacct ggggaaacag aacgtctacg acataggtgt ggagcgcgat 240

cacaactttg ctctgaaaaa tggatttatc gccagcaact gcatctcccg ccgtgcacag 300

ggtgtcacgt tgcaagacct gcctgaaacc gaactgcccg ctgttctgca gccggtcgcg 360

gaggccatgg atgcgatcgc tgcggccgat cttagccaga cgagcgggtt cggcccattc 420

ggaccgcaag gaatcggtca atacactaca tggcgtgatt tcatatgcgc gattgctgat 480

ccccatgtgt atcactggca aactgtgatg gacgacaccg tcagtgcgtc cgtcgcgcag 540

gctctcgatg agctgatgct ttgggccgag gactgccccg aagtccggca cctcgtgcac 600

gcggatttcg gctccaacaa tgtcctgacg gacaatggcc gcataacagc ggtcattgac 660

tggagcgagg cgatgttcgg ggattcccaa tacgaggtcg ccaacatctt cttctggagg 720

ccgtggttgg cttgtatgga gcagcagacg cgctacttcg agcggaggca tccggagctt 780

gcaggatcgc cgcggctccg ggcgtatatg ctccgcattg gtcttgacca actctatcag 840

agcttggttg acggcaattt cgatgatgca gcttgggcgc agggtcgatg cgacgcaatc 900

gtccgatccg gagccgggac tgtcgggcgt acacaaatcg cccgcagaag cgcggccgtc 960

tggaccgatg gctgtgtaga agtactcgcc gatagtggaa accgacgccc cagcactcgt 1020

ccgagggcaa aggaatag 1038

<210> 12
<211> 647
<212> DNA
<213> Homo sapiens

<400> 12
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc ctttcatacg agaccgagat cctgactgtc 300

gagtacggat tgcttcctat cggcaaaatc gtggagaaga ggattgaatg taccgtctat 360

tcagtcgata ataatgggaa catctacaca cagcccgtgg ctcaatggca cgacagagga 420

gagcaggaag tttttgaata ctgtctcgag gacggatccc tcatccgcgc tactaaagat 480

cataagttta tgaccgtgga cggccagatg ctgccaattg acgaaatttt tgaacgagag 540

ctggtgagtt gacctcagta acccaagtga gatcccaggg tgctgggaca ggaggtctgg 600

aagttcctat tccgaagttc ctattctcta gaaagtatag gaacttc 647

<210> 13
<211> 995
<212> DNA
<213> Homo sapiens

<400> 13
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc atctcccgcc gtgcacaggg tgtcacgttg 300

gagtacggat tgcttcctat cggcaaatcc gtggagaaga ggattgaatg taccgtctat 360

tcagtcgata ataatgggaa catctacaca cagcccgtgg ctcaatggca cgacagagga 420

gagcaggaag tttttgaata ctgtctcgag gacggatccc tcatccgcgc tactaaagat 480

cataagttta tgaccgtgga cggccagatg ctgccaattg acgaaatttt tgaacgagag 540

ctggatctga tgagagtcga caaccttcca aacatgagcg agctgattaa ggagaacatg 600

cacatgaagc tgtacatgga gggcaccgtg gacaaccatc acttcaagtg cacatccgag 660

ggcgaaggca agccctacga gggcacccag accatgagaa tcaaggtggt cgagggcggc 720

cctctcccct tcgccttcga catcctggct actagcttcc tctacggcag caagaccttc 780

atcaaccaca cccagggcat ccccgacttc ttcaagcagt ccttccctga gggcttcaca 840

tgggagagag tcaccacata cgaagacggg ggcgtgctga ccgctaccca ggtgagttga 900

cctcagtaac ccaagtgaga tcccagggtg ctgggacagg aggtctggaa gttcctattc 960

cgaagttcct attctctaga aagtatagga acttc 995

<210> 14
<211> 1389
<212> DNA
<213> Homo sapiens

<400> 14
   gaagttccta ttccgaagtt cctattctct agaaagtata ggaacttctc ctggggggct 60
   ccgctggtca ctcacccact ctccctcccc gctcctcgtc cctcctcctc ctcccccctg 120
   ctcctccccc atcatgttct ccaggacacc agcctccagg acggctgcct catctacaac 180
   gtcaagatca gaggggtgaa cttcacatcc aacggccctg tgatgcagaa gaaaacactc 240
   ggctgggagg ccttcaccga gacgctgtac cccgctgacg gcggcctgga aggcagaaac 300
   gacatggccc tgaagctcgt gggcgggagc catctgatcg caaacatcaa gaccacatat 360
   agatccaaga aacccgctaa gaacctcaag atgcctggcg tctactatgt ggactacaga 420
   ctggaaagaa tcaaggaggc caacaacgag acctacgtcg agcagcacga ggtggcagtg 480
   gccagatact gcgacctccc tagcaaactg gggcacaagc ttaatattaa gatcgctacg 540
   cggaagtacc tggggaaaca gaacgtctac gacataggtg tggagcgcga tcacaacttt 600
   gctctgaaaa atggatttat cgccagcaac tgcatctccc gccgtgcaca gggtgtcacg 660
   ttgcaagacc tgcctgaaac cgaactgccc gctgttctgc agccggtcgc ggaggccatg 720
   gatgcgatcg ctgcggccga tcttagccag acgagcgggt tcggcccatt cggaccgcaa 780
   ggaatcggtc aatacactac atggcgtgat ttcatatgcg cgattgctga tccccatgtg 840
   tatcactggc aaactgtgat ggacgacacc gtcagtgcgt ccgtcgcgca ggctctcgat 900
   gagctgatgc tttgggccga ggactgcccc gaagtccggc acctcgtgca cgcggatttc 960
   ggctccaaca atgtcctgac ggacaatggc cgcataacag cggtcattga ctggagcgag 1020
   gcgatgttcg gggattccca atacgaggtc gccaacatct tcttctggag gccgtggttg 1080
   gcttgtatgg agcagcagac gcgctacttc gagcggaggc atccggagct tgcaggatcg 1140
   ccgcggctcc gggcgtatat gctccgcatt ggtcttgacc aactctatca gagcttggtt 1200
   gacggcaatt tcgatgatgc agcttgggcg cagggtcgat gcgacgcaat cgtccgatcc 1260
   ggagccggga ctgtcgggcg tacacaaatc gcccgcagaa gcgcggccgt ctggaccgat 1320
   ggctgtgtag aagtactcgc cgatagtgga aaccgacgcc ccagcactcg tccgagggca 1380
   aaggaatag 1389

<210> 15
<211> 1055
<212> DNA
<213> Homo sapiens

<400> 15
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattta gcgagagcct gacctattgc atctcccgcc gtgcacaggg tgtcacgttg 300

caagacctgc ctgaaaccga actgcccgct gttctgcagc cggtcgcgga ggccatggat 360

gcgatcgctg cggccgatct tagccagacg agcgggttcg gcccattcgg accgcaagga 420

atcggtcaat acactacatg gcgtgatttc atatgcgcga ttgctgatcc ccatgtgtat 480

cactggcaaa ctgtgatgga cgacaccgtc agtgcgtccg tcgcgcaggc tctcgatgag 540

ctgatgcttt gggccgagga ctgccccgaa gtccggcacc tcgtgcacgc ggatttcggc 600

tgtatcagtg gcgactccct gatctcactc gcaatgagcg agctgattaa ggagaacatg 660

cacatgaagc tgtacatgga gggcaccgtg gacaaccatc acttcaagtg cacatccgag 720

ggcgaaggca agccctacga gggcacccag accatgagaa tcaaggtggt cgagggcggc 780

cctctcccct tcgccttcga catcctggct actagcttcc tctacggcag caagaccttc 840

atcaaccaca cccagggcat ccccgacttc ttcaagcagt ccttccctga gggcttcaca 900

tgggagagag tcaccacata cgaagacggg ggcgtgctga ccgctaccca ggtgagttga 960

cctcagtaac ccaagtgaga tcccagggtg ctgggacagg aggtctggaa gttcctattc 1020

cgaagttcct attctctaga aagtatagga acttc 1055

<210> 16
<211> 434
<212> DNA
<213> Homo sapiens

<400> 16
atggtgagca agggcgaggc agtgatcaag gagttcatgc ggttcaaggt gcacatggag 60

ggctccatga acggccacga gttcgagatc gagggcgagg gcgagggccg cccctacgag 120

ggcacccaga ccgccaagct gaaggtgacc aagggtggcc ccctgccctt ctcctgggac 180

atcctgtccc ctcagttcat gtacggctcc agggccttca ccaagcaccc cgccgacatc 240

cccgactact ataagcagtc cttccccgag ggcttcaagt gggagcgcgt gatgaacttc 300

gaggacggcg gcgccgtgac cgtgacccag gtgagttgac ctcagtaacc caagtgagat 360

cccagggtgc tgggacagga ggtctggaag ttcctattcc gaagttccta ttctctagaa 420

agtataggaa cttc 434

<210> 17
<211> 513
<212> DNA
<213> Homo sapiens

<400> 17
   gaagttccta ttccgaagtt cctattctct agaaagtata ggaacttctc ctggggggct 60
   ccgctggtca ctcacccact ctccctcccc gctcctcgtc cctcctcctc ctcccccctg 120
   ctcctccccc atcatgttct ccaggacacc tccctggagg acggcaccct gatctacaag 180
   gtgaagctcc gaggcaccaa cttccctcct gacggccccg taatgcagaa gaagacaatg 240
   ggctgggaag cgtccaccga gcggttgtac cccgaggacg gcgtgctgaa gggcgacatt 300
   aagatggccc tgcgcctgaa ggacggcggc cgctacctgg cggacttcaa gaccacctac 360
   aaggccaaga agcccgtgca gatgcccggc gcctacaacg tagaccgcaa gttggacatc 420
   acctcccaca acgaggacta caccgtggtg gaacagtacg aacgctccga gggccgccac 480
   tccaccggcg gcatggacga gctgtacaag taa 513

<210> 18
<211> 422

<212> DNA
<213> Homo sapiens

<400> 18
atgagcgagc tgattaagga gaacatgcac atgaagctgt acatggaggg caccgtggac 60

aaccatcact tcaagtgcac atccgagggc gaaggcaagc cctacgaggg cacccagacc 120

atgagaatca aggtggtcga gggcggccct ctccccttcg ccttcgacat cctggctact 180

agcttcctct acggcagcaa gaccttcatc aaccacaccc agggcatccc cgacttcttc 240

aagcagtcct tccctgaggg cttcacatgg gagagagtca ccacatacga agacgggggc 300

gtgctgaccg ctacccaggt gagttgacct cagtaaccca agtgagatcc cagggtgctg 360

ggacaggagg tctggaagtt cctattccga agttcctatt cttcaaatag tataggaact 420

tc 422

<210> 19
<211> 528
<212> DNA
<213> Homo sapiens

<400> 19
gaagttccta ttccgaagtt cctattcttc aaatagtata ggaacttctc ctgggggct 60

ccgctggtca ctcacccact ctccctcccc gctcctcgtc cctcctcctc ctcccccctg 120

ctcctccccc atcatgttct ccaggacacc agcctccagg acggctgcct catctacaac 180

gtcaagatca gaggggtgaa cttcacatcc aacggccctg tgatgcagaa gaaaacactc 240

ggctgggagg ccttcaccga gacgctgtac cccgctgacg gcggcctgga aggcagaaac 300

gacatggccc tgaagctcgt gggcgggagc catctgatcg caaacatcaa gaccacatat 360

agatccaaga aacccgctaa gaacctcaag atgcctggcg tctactatgt ggactacaga 420

ctggaaagaa tcaaggaggc caacaacgag acctacgtcg agcagcacga ggtggcagtg 480

gccagatact gcgacctccc tagcaaactg gggcacaaac ttaattaa 528

<210> 20
<211> 104
<212> DNA
<213> Homo sapiens

<400> 20
gtgagttgac ctcagtaacc caagtgagat cccagggtgc tgggacagga ggtctggaag 60

ttcctattcc gaagttccta ttctctagaa agtataggaa cttc 104

<210> 21
<211> 144
<212> DNA

<213> Homo sapiens

<400> 21
gaagttccta ttccgaagtt cctattctct agaaagtata ggaacttctc ctggggggct     60

ccgctggtca ctcacccact ctccctcccc gctcctcgtc cctcctcctc ctcccccctg    120

ctcctccccc atcatgttct ccag                                           144

<210> 22
<211> 104
<212> DNA
<213> Homo sapiens

<400> 22
gtgagttgac ctcagtaacc caagtgagat cccagggtgc tgggacagga ggtctggaag     60

ttcctattcc gaagttccta ttcttcaaat agtataggaa cttc                     104

<210> 23
<211> 144
<212> DNA
<213> Homo sapiens

<400> 23
gaagttccta ttccgaagtt cctattcttc aaatagtata ggaacttctc ctgggggct     60

ccgctggtca ctcacccact ctccctcccc gctcctcgtc cctcctcctc ctcccccctg    120

ctcctccccc atcatgttct ccag                                           144

<210> 24
<211> 573
<212> DNA
<213> Homo sapiens

<400> 24
   atgaaaaagc ctgaactcac cgcgacgtct gtcgagaagt ttctgatcga aaagttcgac     60
   agcgtctccg acctgatgca gctctcggag ggcgaagaat ctcgtgcttt cagcttcgat    120
   gtaggagggc gtggatatgt cctgcgggta aatagctgcg ccgatggttt ctacaaagat    180
   cgttatgttt atcggcactt tgcatcggcc gcgctcccga ttccggaagt gcttgacatt    240
   ggggaattca gcgagagcct gacctattgc ctttcatacg agaccgagat cctgactgtc    300
   gagtacggat tgcttcctat cggcaaaatc gtggagaaga ggattgaatg taccgtctat    360
   tcagtcgata ataatgggaa catctacaca cagcccgtgg ctcaatggca cgacagagga    420
   gagcaggaag tttttgaata ctgtctcgag gacggatccc tcatccgcgc tactaaagat    480
   cataagttta tgaccgtgga cggccagatg ctgccaattg acgaaatttt tgaacgagag    540
   ctggatctga tgagagtcga caaccttcca aac                                 573

<210> 25
<211> 864
<212> DNA
<213> Homo sapiens


<400> 25
attaagatcg ctacgcggaa gtacctgggg aaacagaacg tctacgacat aggtgtggag      60

cgcgatcaca actttgctct gaaaaatgga tttatcgcca gcaactgcat ctcccgccgt     120

gcacagggtg tcacgttgca agacctgcct gaaaccgaac tgcccgctgt tctgcagccg     180

gtcgcggagg ccatggatgc gatcgctgcg gccgatctta gccagacgag cgggttcggc     240

ccattcggac cgcaaggaat cggtcaatac actacatggc gtgatttcat atgcgcgatt     300

gctgatcccc atgtgtatca ctggcaaact gtgatggacg acaccgtcag tgcgtccgtc     360

gcgcaggctc tcgatgagct gatgctttgg gccgaggact gccccgaagt ccggcacctc     420

gtgcacgcgg atttcggctc caacaatgtc ctgacggaca atggccgcat aacagcggtc     480

attgactgga gcgaggcgat gttcggggat tcccaatacg aggtcgccaa catcttcttc     540

tggaggccgt ggttggcttg tatggagcag cagacgcgct acttcgagcg gaggcatccg     600

gagcttgcag gatcgccgcg gctccgggcg tatatgctcc gcattggtct tgaccaactc     660

tatcagagct tggttgacgg caatttcgat gatgcagctt gggcgcaggg tcgatgcgac     720

gcaatcgtcc gatccggagc cgggactgtc gggcgtacac aaatcgcccg cagaagcgcg     780

gccgtctgga ccgatggctg tgtagaagta ctcgccgata gtggaaaccg acgccccagc     840

actcgtccga gggcaaagga atag                                            864


<210> 26
<211> 445
<212> DNA
<213> Homo sapiens


<400> 26
atgaaaaagc ctgaactcac cgcgacgtct gtcgagaagt ttctgatcga aaagttcgac      60

agcgtctccg acctgatgca gctctcggag ggcgaagaat ctcgtgcttt cagcttcgat     120

gtaggagggc gtggatatgt cctgcgggta aatagctgcg ccgatggttt ctacaaagat     180

cgttatgttt atcggcactt tgcatcggcc gcgctcccga ttccggaagt gcttgacatt     240

ggggaattta gcgagagcct gacctattgc ctttcatacg agaccgagat cctgactgtc     300

gagtacggat tgcttcctat cggcaaaatc gtggagaaga ggtgagttga cctcagtaac     360

ccaagtgaga tcccagggtg ctgggacagg aggtctggaa gttcctattc cgaagttcct     420

attctctaga aagtatagga acttc                                           445

<210> 27
<211> 379
<212> DNA
<213> Homo sapiens

<400> 27
gaagttccta ttccgaagtt cctattctct agaaagtata ggaacttctc ctggggggct 60

ccgctggtca ctcacccact ctccctcccc gctcctcgtc cctcctcctc ctcccccctg 120

ctcctccccc atcatgttct ccaggattga atgtaccgtc tattcagtcg ataataatgg 180

gaacatctac acacagcccg tggctcaatg gcacgacaga ggagagcagg aagtttttga 240

atactgtctc gaggacggat ccctcatccg cgctactaaa gatcataagt ttatgaccgt 300

ggacggccag atgctgccaa ttgacgaaat ttttgaacga gagctggatc tgatgagagt 360

cgacaacctt ccaaactga 379

<210> 28
<211> 602
<212> DNA
<213> Homo sapiens

<400> 28
atgattaaga tcgctacgcg gaagtacctg gggaaacaga acgtctacga cataggtgtg 60

gagcgcgatc acaactttgc tctgaaaaat ggatttatcg ccagcaactg catctcccgc 120

cgtgcacagg gtgtcacgtt gcaagacctg cctgaaaccg aactgcccgc tgttctgcag 180

ccggtcgcgg aggccatgga tgcgatcgct gcggccgatc ttagccagac gagcgggttc 240

ggcccattcg gaccgcaagg aatcggtcaa tacactacat ggcgtgattt catatgcgcg 300

attgctgatc cccatgtgta tcactggcaa actgtgatgg acgacaccgt cagtgcgtcc 360

gtcgcgcagg ctctcgatga gctgatgctt tgggccgagg actgccccga agtccggcac 420

ctcgtgcacg cggatttcgg ctccaacaat gtcctgacgg acaatggccg cataacagcg 480

gtcattgact ggagcgaggt gagttgacct cagtaaccca agtgagatcc cagggtgctg 540

ggacaggagg tctggaagtt cctattccga agttcctatt ctctagaaag tataggaact 600

tc 602

<210> 29
<211> 513
<212> DNA
<213> Homo sapiens

<400> 29
gaagttccta ttccgaagtt cctattctct agaaagtata ggaacttctc ctgggggct 60

ccgctggtca ctcacccact ctccctcccc gctcctcgtc cctcctcctc ctcccccctg 120

ctcctccccc atcatgttct ccaggcgatg ttcggggatt cccaatacga ggtcgccaac 180

atcttcttct ggaggccgtg gttggcttgt atggagcagc agacgcgcta cttcgagcgg 240

aggcatccgg agcttgcagg atcgccgcgg ctccgggcgt atatgctccg cattggtctt 300

gaccaactct atcagagctt ggttgacggc aatttcgatg atgcagcttg ggcgcagggt 360

cgatgcgacg caatcgtccg atccggagcc gggactgtcg ggcgtacaca aatcgcccgc 420

agaagcgcgg ccgtctggac cgatggctgt gtagaagtac tcgccgatag tggaaaccga 480

cgccccagca ctcgtccgag ggcaaaggaa tag 513

<210> 30
<211> 995
<212> DNA
<213> Homo sapiens

<400> 30
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc ctttcatacg agaccgagat cctgactgtc 300

gagtacggat tgcttcctat cggcaaaatc gtggagaaga ggattgaatg taccgtctat 360

tcagtcgata ataatgggaa catctacaca cagcccgtgg ctcaatggca cgacagagga 420

gagcaggaag tttttgaata ctgtctcgag gacggatccc tcatccgcgc tactaaagat 480

cataagttta tgaccgtgga cggccagatg ctgccaattg acgaaatttt tgaacgagag 540

ctggatctga tgagagtcga caaccttcca aacatgagcg agctgattaa ggagaacatg 600

cacatgaagc tgtacatgga gggcaccgtg gacaaccatc acttcaagtg cacatccgag 660

ggcgaaggca agccctacga gggcacccag accatgagaa tcaaggtggt cgagggcggc 720

cctctcccct tcgccttcga catcctggct actagcttcc tctacggcag caagaccttc 780

atcaaccaca cccagggcat ccccgacttc ttcaagcagt ccttccctga gggcttcaca 840

tgggagagag tcaccacata cgaagacggg ggcgtgctga ccgctaccca ggtgagttga 900

cctcagtaac ccaagtgaga tcccagggtg ctgggacagg aggtctggaa gttcctattc 960

cgaagttcct attctctaga aagtatagga acttc 995

<210> 31
<211> 1389
<212> DNA
<213> Homo sapiens

<400> 31
   gaagttccta ttccgaagtt cctattctct agaaagtata ggaacttctc ctggggggct 60
   ccgctggtca ctcacccact ctccctcccc gctcctcgtc cctcctcctc ctcccccctg 120
   ctcctccccc atcatgttct ccaggacacc agcctccagg acggctgcct catctacaac 180
   gtcaagatca gaggggtgaa cttcacatcc aacggccctg tgatgcagaa gaaaacactc 240
   ggctgggagg ccttcaccga gacgctgtac cccgctgacg gcggcctgga aggcagaaac 300
   gacatggccc tgaagctcgt gggcgggagc catctgatcg caaacatcaa gaccacatat 360
   agatccaaga aacccgctaa gaacctcaag atgcctggcg tctactatgt ggactacaga 420
   ctggaaagaa tcaaggaggc caacaacgag acctacgtcg agcagcacga ggtggcagtg 480
   gccagatact gcgacctccc tagcaaactg gggcacaagc ttaatattaa gatcgctacg 540
   cggaagtacc tggggaaaca gaacgtctac gacataggtg tggagcgcga tcacaacttt 600
   gctctgaaaa atggatttat cgccagcaac tgcatctccc gccgtgcaca gggtgtcacg 660
   ttgcaagacc tgcctgaaac cgaactgccc gctgttctgc agccggtcgc ggaggccatg 720
   gatgcgatcg ctgcggccga tcttagccag acgagcgggt tcggcccatt cggaccgcaa 780
   ggaatcggtc aatacactac atggcgtgat ttcatatgcg cgattgctga tccccatgtg 840
   tatcactggc aaactgtgat ggacgacacc gtcagtgcgt ccgtcgcgca ggctctcgat 900
   gagctgatgc tttgggccga ggactgcccc gaagtccggc acctcgtgca cgcggatttc 960
   ggctccaaca atgtcctgac ggacaatggc cgcataacag cggtcattga ctggagcgag 1020
   gcgatgttcg gggattccca atacgaggtc gccaacatct tcttctggag gccgtggttg 1080
   gcttgtatgg agcagcagac gcgctacttc gagcggaggc atccggagct tgcaggatcg 1140
   ccgcggctcc gggcgtatat gctccgcatt ggtcttgacc aactctatca gagcttggtt 1200
   gacggcaatt tcgatgatgc agcttgggcg cagggtcgat gcgacgcaat cgtccgatcc 1260
   ggagccggga ctgtcgggcg tacacaaatc gcccgcagaa gcgcggccgt ctggaccgat 1320
   ggctgtgtag aagtactcgc cgatagtgga aaccgacgcc ccagcactcg tccgagggca 1380
   aaggaatag 1389

<210> 32
<211> 705
<212> DNA
<213> Homo sapiens

<400> 32
atgtccaaag gagaagaact gtttaccggt gttgtgccaa ttttggttga actcgatggt 60

gatgtcaacg gacataagtt ctcagtgaga ggcgaaggag aaggtgacgc caccattgga 120

aaattgactc ttaaattcat ctgtactact ggtaaacttc ctgtaccatg gccgactctc 180

gtaacaacgc ttacgtacgg agttcagtgc ttttcgagat acccagacca tatgaaaaga 240

catgactttt ttaagtcggc tatgcctgaa ggttacgtgc aagaaagaac aatttcgttc 300

aaagatgatg gaaaatataa aactagagca gttgttaaat ttgaaggaga tactttggtt 360

aaccgcattg aactgaaagg aacagatttt aaagaagatg gtaatattct tggacacaaa 420

ctcgaataca attttaatag tcataacgta tacatcactg ctgataagca aaagaacgga 480

attaaagcga atttcacagt acgccataat gtagaagatg gcagtgttca acttgccgac 540

cattaccaac aaaacacccc tattggagac ggtccggtac ttcttcctga taatcactac 600

ctctcaacac aaacagtcct gagcaaagat ccaaatgaaa aagggggatc agggggtgaa 660

gttcctattc cgaagttcct attctctaga aagtatagga acttc 705

<210> 33
<211> 114
<212> DNA
<213> Homo sapiens

<400> 33
gaagttccta ttccgaagtt cctattctct agaaagtata ggaacttcgg cggttcaggc 60

ggacgtgacc acatggtcct tcatgagtat gtaaatgctg ctgggattac ataa 114

<210> 34
<211> 687
<212> DNA
<213> Homo sapiens

<400> 34
atggaggagg acaacatggc catcatcaag gagttcatga gattcaaggt gcacatggag 60

ggcagcgtga acggccacga gttcgagatc gagggcgagg gcgagggcca cccctacgag 120

ggcacccaga ccgccaagct gaaggtgacc aagggcggcc ccctgccctt cgcctgggac 180

atcctgagcc cccagttcat gtacggcagc aaggcctacg tgaagcaccc cgccgacatc 240

cccgactacc tgaagctgag cttccccgag ggcttcacct gggagagagt gatgaacttc 300

gaggacggcg gcgtggtgac cgtgacccag gacagcagcc tgcaggacgg ccagttcatc 360

tacaaggtga agctgctggg catcaacttc cccagcgacg gccccgtgat gcagaagaag 420

accatgggct gggaggccag caccgagaga atgtaccccg aggacggcgc cctgaagggc 480

gagatcaacc agagactgaa gctgaaggac ggcggccact acgacgccga ggtgaagacc 540

acctacaagg ccaagaagcc cgtgcagctg cccggcgcct acaacgtgga catcaagctg 600

gacatcacca gccacaacga ggacggggga tcagggggtg aagttcctat tccgaagttc 660

ctattctcta gaaagtatag gaacttc 687

<210> 35
<211> 114
<212> DNA
<213> Homo sapiens

<400> 35
gaagttccta ttccgaagtt cctattctct agaaagtata ggaacttcgg cggttcaggc     60

ggatacacca tcgtggagca gtacgagaga gccgaggcca gacacagcac ctaa          114

<210> 36
<211> 702
<212> DNA
<213> Homo sapiens

<400> 36
atggtgagca agggtgagga ggataacatg gcctctctcc cagcgactca tgagttacac     60

atctttggct ccatcaacgg tgtggacttt gacatggtgg gtcagggtac cggcaatcca    120

aatgatggtt atgaggagtt aaacctgaag tccaccaagg gtgacctcca gttctccccc    180

tggattctgg tccctcatat cgggtatggc ttccatcagt acctgcccta ccctgacggg    240

atgtcgcctt tccaggccgc catggtagat ggctccggat accaagtcca tcgcacaatg    300

cagtttgaag atggtgcctc ccttactgtt aactaccgct acacctacga gggaagccac    360

atcaaaggag aggcccaggt gatggggact ggtttccctg ctgacggtcc tgtgatgacc    420

aacacgctga ccgctgcgga ctggtgcatg tcgaagaaga cttaccccaa cgacaaaacc    480

atcatcagta cctttaagtg gagttacacc actgtaaatg gcaaacgcta ccggagcact    540

gcgcggacca cctacacctt tgccaagcca atggcggcta actatctgaa gaaccagccg    600

atgtacgtgt tccgtaagac ggagctcaag cactccatgg ggggatcagg gggtgaagtt    660

cctattccga agttcctatt ctctagaaag tataggaact tc                       702

<210> 37
<211> 114
<212> DNA
<213> Homo sapiens

<400> 37
gaagttccta ttccgaagtt cctattctct agaaagtata ggaacttcgg cggttcaggc     60

ggaaccgagc tcaacttcaa ggagtggcaa aaggccttta ccgatatgat gtaa          114

<210> 38
<211> 135
<212> DNA
<213> Homo sapiens

<400> 38
atgaccatgg acctgcctga cgaccactac ctgtccaccc agaccatcct gtccaaggac 60

ctgaacggtg ggggatcagg gggtggcgaa gttcctattc cgaagttcct attctctaga 120

aagtatagga acttc 135

<210> 39
<211> 135
<212> DNA
<213> Homo sapiens

<400> 39
gaagttccta ttccgaagtt cctattctct agaaagtata ggaacttcgg tggaggcagc 60

gggggcggtg aaaagcgaga ccatatggtt ttgcttgagt atgttacagc ggctggcatt 120

accgatgcat cataa 135

<210> 40
<211> 591
<212> DNA
<213> Homo sapiens

<400> 40
atgcgcaagg gcgaggagct gttcaccggc gtggtgccca tcctgatcga gctggacggc 60

gacgtgaacg gccacaagtt cttcgtgcgc ggcgagggcg agggcgacgc caccatcggc 120

aagctgtccc tgaagttcat ctgcaccacc ggcaagctgc ccgtgccctg gcccaccctg 180

gtgaccaccc tgacctacgg cgtgcagtgc ttctcccgct accccgacca catgaagcgc 240

cacgacttct tcaagtccgc catgcccgag ggctacgtgc aggagcgcac catctacttc 300

aaggacgacg gcacctacaa gacccgcgcc gaggtgaagt tcgagggcga caccctggtg 360

aaccgcatcg agctgaaggg catcgacttc aaggaggacg gcaacatcct gggccacaag 420

ctggagtaca acttcaactc ccacaaggtg tacatcaccg ccgacaagca gaacaacggc 480

atcaaggcca acttcaccat ccgccacaac gtggaggacg gctccgtgca gctggccgac 540

cactaccagc agaacacccc catcggcgac ggccccgtgc tgctgcccta a 591